# FASCICULE DE BREVET EUROPEEN

(11) **EP 2 333 018 B1**
(45) Date de publication et mention de la délivrance du brevet: **22.11.2017**
(21) Numéro de dépôt: 11154385.6
(22) Date de dépôt: 13.10.2005
(51) Int. Cl.: C09B 35/32, C07C 321/16, C07D 251/50, C07D 251/52

(54) **Composition de teinture comprenant un colorant disulfure particulier et procédé de coloration des fibres keratiniques humaines à partir de ce colorant**
Färbezusammensetzung enthaltend einen Disulfid Farbstoff und Verfahren zum Färben von Keratinfasern mit dieser Färbezusammensetzung
Dying composition comprising a particular disulfide dye and process of dying keratinous fibres with this dye

(30) Priorité: 14.10.2004 FR 0410864
(43) Date de publication de la demande: 15.06.2011
(62) Demande divisionnaire de: 05292159.0
(73) Titulaire: L'Oréal, 75008 Paris (FR)
(72) Inventeur: Daubresse, Nicolas, 78170 La Celle Saint Cloud (FR); Genain, Gilles, 75116 Paris (FR)
(74) Mandataire: Wattremez, Catherine

(56) Documents cités:
- EP-A- 0 271 322
- WO-A1-03/099242
- WO-A2-2005/097051
- FR-A- 1 156 407
- FR-A- 2 822 696
- FR-A- 2 825 624
- LEWIS D M ET AL: "THE ROLE OF VINYLSULPHONYL REACTIVE DYES IN PREVENTION OF WOOL DAMAGE", 1 octobre 1991 (1991-10-01), JOURNAL OF THE SOCIETY OF DYERS AND COLOURISTS, SOCIETY OF DYERS AND COLOURISTS. BRADFORD, GB, PAGE(S) 357-362, XP000244313, ISSN: 0037-9859 * le document en entier *
- OKAWA H ET AL: "SYNTHESIS AND CHARACTERIZATION OF AN ALKANETHIOL THIN FILM CONTAINING A HEMICYANINE DYE", MOLECULAR CRYSTALS AND LIQUID CRYSTALS SCIENCE AND TECHNOLOGY. SECTION A. MOLECULAR CRYSTALS AND LIQUID CRYSTALS, GORDON AND BREACH PUBLISHERS, CH, CH, vol. 377, 1 janvier 2002 (2002-01-01), pages 137-140, XP008056025, ISSN: 1058-725X, DOI: DOI:10.1080/10587250211691
- ASHWELL G J ET AL: "Molecular Rectification: Self-Assembled Monolayers in which Donor-(pi-bridge)-Acceptor Moieties are centrally located and symmetrically coupled to both Gold Electrodes", JOURNAL OF THE AMERICAN CHEMICAL SOCIETY, AMERICAN CHEMICAL SOCIETY, US, vol. 126, no. 22, 1 janvier 2004 (2004-01-01), pages 7102-7110, XP002487356, ISSN: 0002-7863, DOI: DOI:10.1021/JA049633U

## Description

L'invention a pour objet une composition de teinture comprenant un colorant disulfure particulier ainsi qu'un procédé de coloration des fibres kératiniques en particulier humaines, et notamment les cheveux à partir de cette composition. L'invention a aussi pour objet des colorants disulfure nouveaux.

Il est connu de teindre les fibres kératiniques, notamment humaines, par une coloration directe. Le procédé classiquement utilisé en coloration directe consiste à appliquer sur les fibres kératiniques des colorants directs qui sont des molécules colorées et colorantes ayant une affinité pour les fibres, à les laisser diffuser, puis à rincer les fibres.

Les colorants directs qui sont classiquement utilisés sont par exemple des colorants du type nitrés benzéniques, des colorants anthraquinoniques, des nitropyridines, des colorants du type azoïque, xanthénique, acridinique, azinique ou triarylméthane.

Les colorations qui résultent de l'utilisation de colorants directs sont des colorations temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Il est aussi connu d'obtenir des colorations permanentes avec des compositions tinctoriales contenant des précurseurs de colorant d'oxydation, appelés généralement bases d'oxydation, tels que des ortho ou para-phénylènediamines, des ortho ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues en associant ces bases d'oxydation à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs, permet l'obtention d'une riche palette de couleurs.

Ce procédé de coloration d'oxydation consiste à appliquer sur les fibres kératiniques, des bases ou un mélange de bases et de coupleurs avec de l'eau oxygénée à titre d'agent oxydant, à laisser diffuser, puis à rincer les fibres. Les colorations qui en résultent sont permanentes, puissantes, et résistantes aux agents extérieurs, notamment à la lumière, aux intempéries, aux lavages, à la transpiration et aux frottements.

Les systèmes de coloration d'oxydation permettent d'obtenir des colorations de fond relativement tenaces au shampooing mais ne permettent pas d'obtenir des nuances chromatiques.

Pour augmenter la ténacité des colorations directes, il est connu de fixer des colorants directs par liaison covalente au cheveu. Par exemple, il est connu de faire réagir des colorants à groupements réactifs avec les résidus de cystine ou de cystéine très nombreux dans les fibres capillaires. Sont ainsi décrits certains colorants portant des fonctions sels de Bunte et isothiuroniums, ou d'autres groupements protecteurs de thiols. Cependant, l'obtention de la forme réactive du colorant nécessite en général l'utilisation de milieux fortement basiques. De plus, les fonctions thiols sont généralement générées en excès ce qui rend nécessaire une étape de post neutralisation à la suite de la coloration.

D'autres colorants disulfure connus pour la coloration des fibres kératiniques sont des dérivés disulfure de dérivés d'aminothiophénol. De tels colorants sont décrits par exemple dans le brevet FR 1156407. Ces colorants peuvent être utilisés dans des conditions relativement douces, en présence d'un milieu légèrement réducteur ou après un pré-traitement réducteur du cheveu. Cependant, ces colorants peuvent occasionner des virages de couleur lors de l'application.

Par ailleurs, il est connu dans l'article « The role of the vinylsulphonyl reactive dyes in prevention of wool damage », J. Soc. Dyers Colourists, vol. 107, octobre 1991, p. 357-362 de colorer la laine à chaud (100°C) par un colorant disulfure, le groupement disulfure servant à générer un groupement vinylsulphonyle, afin de réduire la dégradation des fibres de laine.

Le but de la présente invention est de fournir de nouveaux systèmes de coloration des fibres kératiniques, notamment humaines, en particulier les cheveux, qui ne présentent pas les inconvénients des colorants directs existants. En particulier, un des buts de la présente invention est de fournir des systèmes de coloration directe permettant d'obtenir des colorations chromatiques, très tenaces, notamment face à des shampooings successifs.

Ce but est atteint avec la présente invention qui a pour objet un procédé de coloration des fibres kératiniques humaines consistant à appliquer sur les fibres, une composition tinctoriale, comprenant dans un milieu cosmétique approprié, au moins un colorant disulfure choisi parmi les colorants de formules (I), (II), (III) ou (IV) suivantes : A- (X)ₚ - Cₛₐₜ - S-S - Cₛₐₜ - (X)ₚ - A (I) 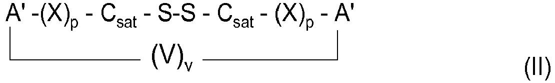 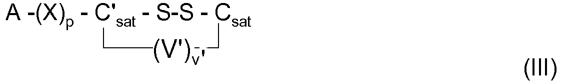 A- (X)ₚ - Cₛₐₜ - S-S - Cₛₐₜ - (X)ₚ - D (IV) leurs sels, solvates tels que hydrates, formules dans lesquelles :
- A, et A', identiques ou différents, représentent un radical contenant au moins un chromophore issu de colorants de type cyanines, lesdits chromophores A et/ou A' contiennent un radical cationique porté par ou inclus dans au moins un des chromophores, ledit radical cationique est un ammonium quaternaire ;
- V et V', identiques ou différents représentent un groupe pontant
- v et v', identiques ou différents représentent 0 ou 1 ;
- X, identiques ou différents, représentent une chaîne hydrocarbonée en C₁-C₃₀, linéaire ou ramifiée, saturée ou insaturée, éventuellement interrompue et/ ou éventuellement terminée à l'une ou deux de ses extrémités par un ou plusieurs groupes divalents ou leur combinaisons choisis parmi :
   - -N(R)-, -N⁺(R)(R)-, -O-, -S-, -CO-, -SO₂- avec R, identiques ou différents, choisi parmi un hydrogène, un radical alkyle en C1-C4, hydroxyalkyle, aminoalkyle
   - un radical (hétéro)cyclique aromatique ou non, saturé ou insaturé, condensé ou non comprenant éventuellement un ou plusieurs hétéroatomes identiques ou non, éventuellement substitué ;
- le coefficient p est égal à 0 ou 1 ;
- Cₛₐₜ, C'ₛₐₜ, identiques ou différents représentent une chaîne alkylène en C₁-C₁₈, linéaire ou ramifiée, éventuellement substituée, éventuellement cyclique ;
- D correspond à un radical choisi parmi les radicaux hydroxy, hydroxyalkyle, alcoxy, carboxyliques, carboxylates, amino, alkylamino, dialkylamino.

Le procédé de l'invention permet d'accéder à des couleurs chromatiques ou à des couleurs de fond très tenaces aux shampooings, aux agressions courantes (soleil, transpiration), et aux autres traitements capillaires.

L'invention a aussi pour objet une composition tinctoriale comprenant, dans un milieu cosmétique approprié pour la teinture des fibres kératiniques notamment humaines, au moins un colorant disulfure choisi parmi les colorants de formules (I), (II), (III) ou (IV).

Un autre objet de l'invention est l'utilisation d'au moins un colorant disulfure de formule (I), (II), (III) ou (IV), pour la teinture des fibres kératiniques, notamment humaines, en particulier les cheveux.

L'invention a enfin pour objet les colorants disulfure de formules (I), (II) (III) ou (IV), en tant que tels, leurs sels, et leurs solvates.

Au sens de la présente invention, et à moins qu'une indication différente ne soit donnée :
- les radicaux aryles ou hétéroaryles ou la partie aryle ou hétéroaryle d'un radical peuvent être substitués par au moins un substituant porté par un atome de carbone, choisi parmi :
   - un radical alkyle en C₁-C₁₆, de préférence en C₁-C₈, éventuellement substitué par un ou plusieurs radicaux choisis parmi les radicaux hydroxy, alcoxy en C₁-C₂, (poly)-hydroxyalcoxy en C₂-C₄, acylamino, amino substitué par deux radicaux alkyle, identiques ou différents, en C₁-C₄, éventuellement porteurs d'au moins un groupement hydroxyle ou, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué comprenant éventuellement un autre hétéroatome identique ou différent de l'azote ;
   - un atome d'halogène tel que chlore, fluor ou brome ;
   - un groupement hydroxyle ;
   - un radical alcoxy en C₁-C₂ ; un radical (poly)-hydroxyalcoxy en C₂-C₄ ;
   - un radical amino ; un radical amino substitué par un ou deux radicaux alkyle, identiques ou différents, en C₁-C₄ éventuellement porteurs d'au moins un groupement hydroxyle ou amino, par deux radicaux alkyle en C₁-C₃ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
   - un radical acylamino (-NR-COR') dans lequel le radical R est un atome d'hydrogène, un radical alkyle en C₁-C₄ éventuellement porteur d'au moins un groupement hydroxyle et le radical R' est un radical alkyle en C₁-C₂ ; un radical carbamoyle ((R)₂N-CO-) dans lequel les radicaux R, identiques ou non, représentent un atome d'hydrogène, un radical alkyle en C₁-C₄ éventuellement porteur d'au moins un groupement hydroxyle ; un radical alkylsulfonylamino (R'SO₂-NR-) dans lequel le radical R représente un atome d'hydrogène, un radical alkyle en C₁-C₄ éventuellement porteur d'au moins un groupement hydroxyle et le radical R' représente un radical alkyle en C₁-C₄, un radical phényle ; un radical aminosulfonyle ((R)₂N-SO₂-) dans lequel les radicaux R, identiques ou non, représentent un atome d'hydrogène, un radical alkyle en C₁-C₄ éventuellement porteur d'au moins un groupement hydroxyle,
   - un radical carboxylique sous forme acide ou salifiée (de préférence avec un métal alcalin ou un ammonium, substitué ou non) ;
   - un radical nitro ;
   - un groupement nitrile (CN) ;
   - un groupement trifluorométhyle(CF₃) ;
- la partie cyclique ou hétérocyclique d'un radical non aromatique peut être substituée par au moins un substituant porté par un atome de carbone choisi parmi les groupements :
   - hydroxyle,
   - alcoxy en C₁-C₄, (poly)hydroxyalcoxy en C₂-C₄,
   - alkylcarbonylamino ((RCO-NR'-) dans lequel le radical R' est un atome d'hydrogène, un radical alkyle en C₁-C₄ éventuellement porteur d'au moins un groupement hydroxyle et le radical R est un radical alkyle en C₁-C₂, amino substitué par deux groupements alkyle identiques ou différents en C₁-C₄ éventuellement porteurs d'au moins un groupement hydroxyle, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
- une chaîne hydrocarbonée est insaturée lorsqu'elle comporte une ou plusieurs liaisons doubles et/ou une ou plusieurs liaisons triples ;
- un radical hétéroaromatique ou hétéroaryle, correspond à un radical aromatique dans lequel au moins l'un des atomes de carbone est remplacé par un hétéroatome, choisi parmi l'azote, l'oxygène ou le soufre.

De plus, sauf indication contraire, les bornes délimitant l'étendue d'une plage de valeurs sont comprises dans cette plage de valeurs.

Selon la présente invention, on entend par « chromophore » un radical issu d'un colorant, c'est-à-dire un radical issu d'une molécule absorbant dans le domaine visible du rayonnement (entre 400 et 800 nm).

Les radicaux A et A' des formules (I), (II), (III) et (IV) peuvent contenir un ou plusieurs chromophores, identiques ou différents.

Au sens de la présente invention, les chromophores sont dits différents lorsqu'ils diffèrent par leur structure chimique. De tels chromophores peuvent être des chromophores issus de familles différentes ou d'une même famille à la condition de présenter des structures chimiques différentes.

Les chromophores utiles dans la présente invention, sont des radicaux issus des colorants cyanines (comme les azacarbocyanines, diazacarbocyanines, diazahémicyanines, hémicyanines, tétraazacarbocyanines).

On peut aussi citer les chromophores décrits dans les documents US 5888252, EP 1133975, WO 03/029359, EP 860636, WO 95/01772, WO 95/15144, EP 714954. On peut aussi citer ceux listés dans l'encyclopédie "The chemistry of synthetic dye" de K. VENKATARAMAN, 1952, Academic press vol 1 à 7, dans l'encyclopédie "Kirk Othmer" "Chemical technology", chapitre "dyes and Dye intermediate", 1993, Wiley and sons, et dans divers chapitre de l'encyclopédie "ULLMANN's ENCYCLOPEDIA of Industrial chemistry" 7th édition, Wiley and sons.

De préférence, le radical cationique est un ammonium quaternaire.

Ces radicaux cationiques sont par exemple un radical alkylammonium, acridinium, benzimidazolium, benzobistriazolium, benzopyrazolium, benzopyridazinium, benzoquinolium, benzothiazolium, benzotriazolium, benzoxazolium, bi-pyridinium, bis-tetrazolium, dihydrothiazolium, imidazopyridinium, imidazolium, indolium, isoquinolium, naphthoimidazolium, naphthooxazolium, naphthopyrazolium, oxadiazolium, oxazolium, oxazolopyridinium, oxonium, phénazinium, phénooxazolium, pyrazinium, pyrazolium, pyrazoyltriazolium, pyridinium, pyridinoimidazolium, pyrrolium, quinolium, tétrazolium, thiadiazolium, thiazolium, thiazolopyridinium, thiazoylimidazolium, thiopyrylium, triazolium, xanthylium.

Des exemples de chromophores cationiques utiles dans la présente invention ont été cités précédemment. D'autres exemples sont donnés dans les demandes de brevet WO 95/01772, WO 95/15144, EP 714954, EP 318294, WO 03/029359.

De préférence, dans le cas des formules (I), (II) et (IV), Cₛₐₜ représente une chaîne -(CH₂)ₙ- avec n entier, compris entre 1 et 8.

De préférence, dans le cas de la formule (III), Cₛₐₜ représente un radical -(CH₂)ₙ-, C'ₛₐₜ représente un radical 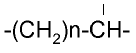 n ayant la même signification que précédemment

Conformément à un mode de réalisation particulier de l'invention, dans les formules (I), (II), (III) ou (IV) précitées, lorsque p est égal à 1, X représente la séquence suivante :

-(T)ₜ-(Y)_{y}-(Z)_{z}-

ladite séquence étant reliée dans les formules (I), (II), (III) ou (IV), comme suit :
- Cₛₐₜ (ou C'ₛₐₜ)-(T)ₜ-(Y)_{y}-(Z)_{z}-(A ou A') ; dans laquelle
   **T** représente un ou plusieurs radicaux ou leurs combinaisons choisis parmi -SO₂-, -O-, -S-, -N(R)-, -N+(R)(R) -CO-, R représentant un atome d'hydrogène, un radical alkyle en C₁-C₄, ou hydroxyalkyle en C1-C4;
   le coefficient **t** vaut 0 ou 1 ;
   **Y** représente :
      - un radical choisi parmi -(CH₂)₂-SO₂- ; -CH₂-CHR-CO-NR'- avec R, R', identiques ou non, représentant un atome d'hydrogène, un radical alkyle en C₁-C₄.
      - un groupement de formule (a), (a') ou (a") : 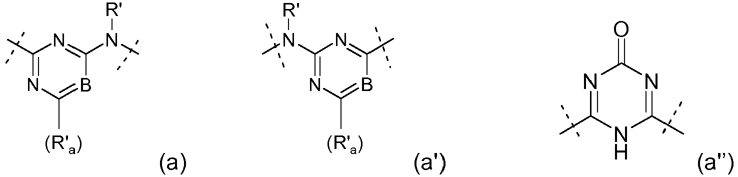 dans laquelle
         - B représente -N-, -CRₐ, avec Rₐ représentant un atome d'hydrogène, un atome d'halogène choisi parmi le chlore ou le fluor, un groupement nitro, un groupement pyridinium éventuellement substitué;
         - R' a la même définition que précédemment
         - R'ₐ représente :
            - un atome d'hydrogène
            - un atome de chlore ou un atome de fluor
            - un groupement pyridinium éventuellement substitué par au moins un groupement R_{c}, R_{c} prouvant être un groupement alkyle en C₁-C₄, un atome d'halogène, un groupement carboxylique -COOM, (avec M représentant un atome d'hydrogène, un métal alcalin, un groupement ammonium ou un groupement ammonium substitué par un ou plusieurs radicaux alkyle, identiques ou non, linéaires ou ramifiés, en C1-C18, éventuellement porteurs d'au moins un hydroxyle) ; un groupement ester -COOR_{d} avec R_{d} représentant un radical alkyle en C₁-C₄ ; un groupement amide -CON(R_{d})₂ avec R_{d} identiques ou non, représentant un atome d'hydrogène ou un radical alkyle en C₁-C₄ ;
            - un groupement hydroxyle
            - un groupement amino, alkylamino ou dialkylamino, les groupements alkyles identiques ou différents, en C1-C18, étant linéaires ou ramifiés, éventuellement interrompus par un hétéroatome choisi parmi N, O, éventuellement substitués par un ou plusieurs groupements hydroxyles
            - un groupement NHNHCOR où R représente un groupement alkyle linéaire ou ramifié en C1-C10
      - un groupement de formule (b) suivante : 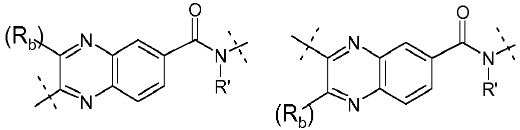 dans laquelle
         - R' a la même définition que précédemment
         - R_{b} représente
            - un atome de chlore
            - un groupement amino, alkylamino ou dialkylamino, les groupements alkyles identiques ou différents, en C₁-C₁₈, étant linéaires ou ramifiés, éventuellement interrompus par un hétéroatome choisi parmi N, O, S, éventuellement substitués par un ou plusieurs hydroxyles
            - un hétérocycle azoté, saturé ou insaturé, pouvant être substitué
            - un groupement arylamino, dans lequel de préférence le radical aryle est en C₆ ;
            **y** vaut 0 ou 1 ;
            **Z** représente
            - -(CH₂)ₘ- avec m entier compris entre 1 et 8
            - -(CH₂CH₂O)_{q}- ou -(OCH₂CH₂)_{q}- dans lesquelles q est un entier compris entre 1 et 15
            - un radical aryle, alkylaryle ou arylalkyle dont le radical alkyle est en C₁-C₄ et le radical aryle est de préférence en C₆, étant éventuellement substitué par au moins un groupement SO₃M avec M représentant un atome d'hydrogène, un métal alcalin ou un groupement ammonium ammonium substitué par un ou plusieurs radicaux alkyle, identiques ou non, linéaires ou ramifiés, en C1-C18 éventuellement porteurs d'au moins un hydroxyle
            **z** vaut 0 ou 1.

Avantageusement, Y représente l'un des groupements ci-dessous, 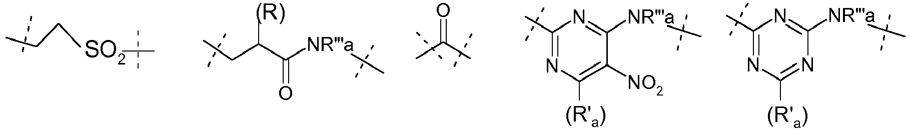 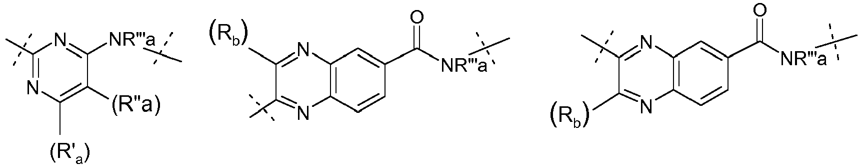 dans lesquels les radicaux R, R'ₐ et R_{b} sont définis comme précédemment ; R"ₐ, a la même définition que R'ₐ, indépendamment les uns des autres ; R'''ₐ représente un atome d'hydrogène ou un radical alkyle.

De préférence, Y représente les groupements suivants : 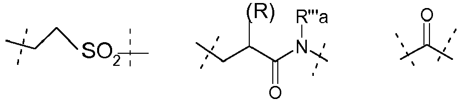 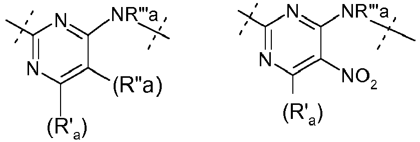 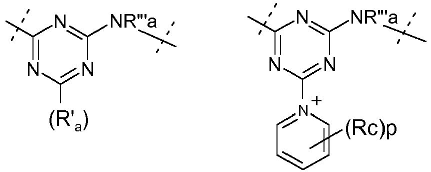 dans lesquels
- R représente un atome d'hydrogène ou un radical méthyle
- R'ₐ, R"ₐ, identiques ou non, représentent un atome de chlore, de fluor ou d'hydrogène
- R_{c} représente un radical alkyle en C₁-C₄ ; un groupement carboxylique -COOM avec M représentant un atome d'hydrogène, un métal alcalin, un groupement ammonium substitué ou non ; un groupement ester -COOR_{d} avec R_{d} représentant un radical alkyle en C₁-C₂, ou un groupement amide -CON(R_{d})₂ avec R_{d}, identiques ou non, représentant un atome d'hydrogène, un radical alkyle en C₁-C₂
- R'''ₐ représentant un atome d'hydrogène, un radical alkyle avantageusement en C₁-C₂ et p est compris entre 0 et 2.

Par ailleurs, selon un mode de réalisation particulier de l'invention, Z représente :

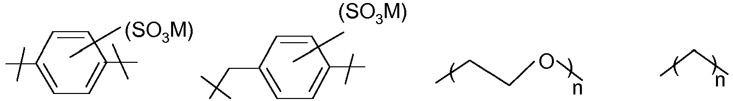

Comme indiqué auparavant, dans la formule (II), V représente un groupe pontant les deux radicaux A' identiques ou différents et v peut être égal à 0 ou 1.

Dans cette variante, le groupe V pontant les deux chromophores A' représente un radical alkyle en C₁-C₈, éventuellement terminé à l'une ou ses deux extrémités par un groupement choisi parmi amine, amide ou ester.

Conformément à un mode de réalisation particulier de l'invention, le colorant disulfure est tel que v est égal à 0.

Conformément à un mode préféré de l'invention, les deux radicaux contenant au moins un chromophore A et A' contiennent au moins un radical cationique porté ou inclus dans ou sur un chromophore, à titre d'exemple on peut citer les composés de formule suivante, leurs sels, hydrates ou solvates :

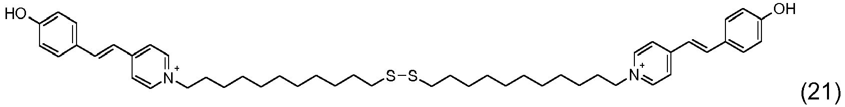

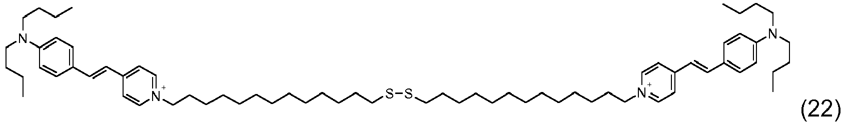

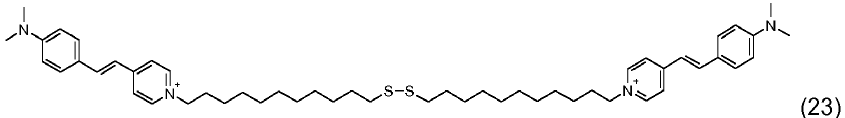

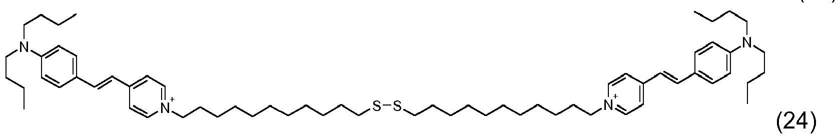

Ces quatre composés, indépendamment de tout choix de forme libre ou salifiée, ainsi que leur mode de préparation sont connus de la technique.

Plus particulièrement, ledit sel d'acide organique ou minéral est choisi parmi les chlorhydrates, les bromhydrates, les sulfates, parmi lesquels le méthylsulfate et l'éthylsulfate, les citrates, les succinates, les tartrates, les lactates, les acétates, les méthosulfates, les tosylates, les benzènesulfonates, les phosphates et les acétates.

Les colorants disulfure peuvent être préparés selon des méthodes connues de l'homme de l'art.

Selon une première possibilité, on peut faire réagir un composé disulfure comprenant deux fonctions amine, de préférence primaire ou secondaire avec une quantité suffisante d'un "chromophore réactif" ou d'un composé comprenant un tel "chromophore réactif", en d'autres termes comprenant une fonction électrophile.

Parmi les "chromophores réactifs", on peut citer les colorants réactifs répertoriés comme tels dans Colour Index et comportant notamment une fonction vinylsulfone, sulfatoéthylsulfone, mono- di- chlorotriazine, mono- di-chloro pyrimidine, difluoro chloro pyrimidine, dichloroquinoxaline, bromo-vinylsulfone.

Conviennent aussi, en tant que chromophores réactifs, les composés chromophores comprenant au moins un groupement susceptible de réagir avec une fonction amine pour donner un groupement sulfamide (-SO₂-N-), amide (-CO-N-). Par exemple, on peut mentionner les groupes -SO₃M', -COO M' (avec M' représentant un atome d'hydrogène, un métal alcalin, comme le sodium, le potassium, un groupement ammonium, un groupement ammonium substitué par un ou plusieurs groupement alkyle, identiques ou non, linéaires ou ramifiés, en C1-C10, éventuellement porteurs d'au moins un hydroxyle), que l'on peut activer préalablement, selon des méthodes connues, respectivement en groupement -SO₂Cl, -COCl.

Il est ainsi envisageable de mettre en oeuvre, à titre de chromophore réactif, les colorants acides du Colour Index répertoriés comme tels.

On pourra se référer notamment à l'ouvrage Advanced Organic Chemistry, March, 4ème Ed, pour avoir plus de détails sur les conditions opératoires mises en oeuvre.

Toujours dans le cadre de cette première possibilité, on peut mettre en oeuvre des chromophores comprenant un groupement labile directement lié ou non au chromophore et susceptible d'être substitué par un groupement amine, tel que Cl, Br, F, O-alkyle (par exemple O-Me), O-aryle, O-alkylaryle (par exemple O-benzyle).

Les colorants disulfure peuvent aussi être obtenus, dans le cadre de cette possibilité, en utilisant des chromophores possédant une fonction acrylate (-OCO-C=C-) sur laquelle on effectue une réaction d'addition.

Conformément à une autre possibilité, les colorants disulfures peuvent être obtenus en faisant réagir un composé disulfure avec un composé portant deux fonctions acide carboxylique activées selon les méthodes classiques (par exemple réaction avec un carbodiimide ou avec le chlorure de thionyle). Le produit résultant est ensuite mis à réagir avec un chromophore porteur d'une fonction nucléophile, par exemple de type amine primaire ou secondaire, ou de type alcool aliphatique ou aromatique comme le phénol.

Là encore, on pourra se référer à l'ouvrage Advanced Organic Chemistry, March, 4ème Ed, pour avoir plus de détails sur les conditions opératoires mises en oeuvre.

Conformément à une troisième possibilité, les colorants disulfure peuvent être obtenus par réaction d'un composé comprenant un groupement disulfure et deux groupements hydroxyle activés préalablement en groupements partants (par exemple mésylate, tosylate) avec un chromophore portant une fonction nucléophile, avantageusement de type amine primaire,secondaire ou tertiaire, hétéroaromatique ou non, par exemple de type pyridine, imidazole, benzimidazole.

Conformément à une quatrième possibilité, les colorants disulfure peuvent être obtenus par oxydation ménagée de colorants portant une fonction SH.

Conformément à une cinquième possibilité, et notamment pour la préparation des composés répondant aux formules (III) et (IV), les colorants disulfures peuvent être obtenus par une variante des possibilités une, deux ou trois décrites ci-dessus, en utilisant une quantité molaire de réactif disulfure supérieure ou égale à la quantité molaire de réactif contenant le groupement chromophore.

La préparation des colorants disulfure répondant à la formule (I) est en revanche facilitée par l'emploi d'une quantité molaire de reactif contenant le groupement chromophore de préférence supérieure ou égale à deux fois la quantité de reactif disulfure.

Conformément à une sixième possibilité, et notamment pour la préparation des composés répondant à la formule (I) et dont les deux groupements A, d'une part, X d'autre part, sont différents, les composés disulfures peuvent être obtenus à partir de composés disulfures répondant à la formule (IV)

La composition tinctoriale utile dans le procédé de l'invention peut contenir un ou plusieurs colorants disulfure de formules (I), (II), (III) ou (IV).

La composition contient en général une quantité de colorant disulfure comprise entre 0,001 et 50% par rapport au poids total de la composition. De préférence, cette quantité est comprise entre 0,005 et 20% en poids et encore plus préférentiellement entre 0,01 et 5% en poids par rapport au poids total de la composition.

La composition tinctoriale peut en outre contenir des colorants directs différents des colorants disulfure de formules (I), (II), (III) ou (IV). Ces colorants directs sont par exemple choisis parmi les colorants directs listés auparavant, et notamment parmi les colorants directs nitrés benzéniques neutres, acides ou cationiques, les colorants directs azoïques neutres, acides ou cationiques, les colorants tétraazapentaméthiniques, les colorants quinoniques et en particulier anthraquinoniques neutres, acides ou cationiques, les colorants directs aziniques, les colorants directs triarylméthaniques, les colorants directs indoaminiques et les colorants directs naturels.

Parmi les colorants de type tétraazapentaméthiniques utilisables, on peut citer les composés suivants figurant dans le tableau ci-dessous, An représentant en général un anion organique ou minéral comme défini précédemment :

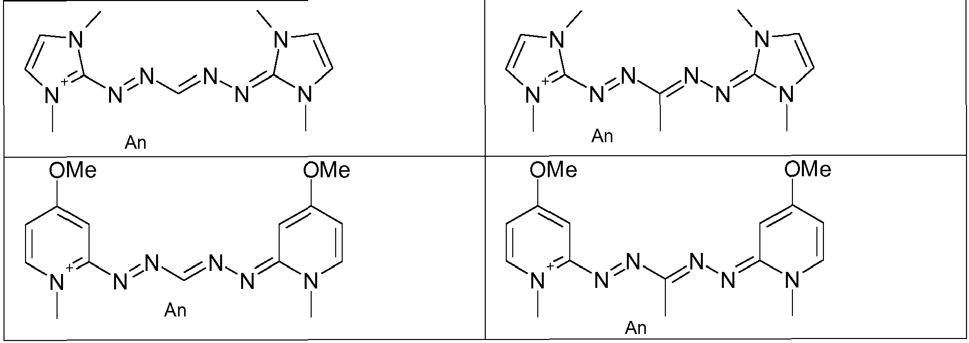

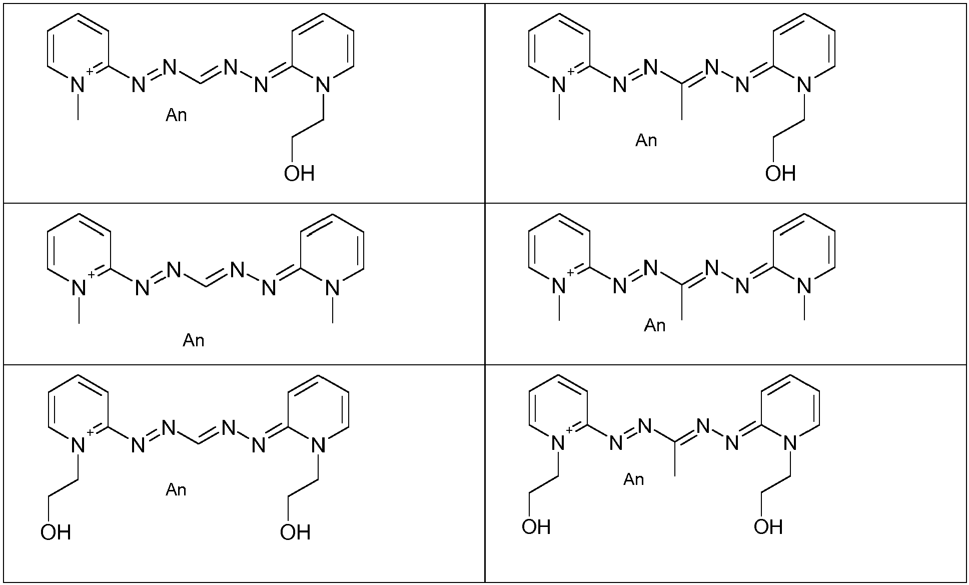

Parmi les colorants directs naturels, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

La composition tinctoriale peut contenir une ou plusieurs base d'oxydation et/ou un ou plusieurs coupleurs conventionnellement utilisés pour la teinture de fibres kératiniques.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition

Parmi ces coupleurs, on peut notamment citer les méta-phénylènediamines, les méta-aminophénols, les méta-diphénols, les coupleurs naphtaléniques, les coupleurs hétérocycliques et leur sels d'addition.

Le ou les coupleurs sont chacun généralement présents en quantité comprise entre 0,001 et 10 % en poids du poids total de la composition tinctoriale, de préférence entre 0,005 et 6 %.

La ou les bases d'oxydation présentes dans la composition tinctoriale sont en général présentes chacune en quantité comprise entre 0,001 à 10 % en poids du poids total de la composition tinctoriale, de préférence entre 0,005 et 6 % en poids.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que les hydroxydes de métal alcalin comme la soude, la potasse, l'ammoniaque, les amines ou les alcanolamines.

Le milieu approprié pour la teinture, appelé aussi support de teinture, est un milieu cosmétique généralement constitué par de l'eau ou par un mélange d'eau et d'au moins un solvant organique. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants lorsqu'ils sont présents sont, de préférence présents dans des proportions de préférence comprises entre 1 et 40 % en poids environ par rapport au poids total de la composition tinctoriale, et encore plus préférentiellement entre 5 et 30 % en poids environ.

La composition tinctoriale peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

La composition peut aussi comprendre au moins un autre composé disulfure additionnel différent de celui correspondant aux formules (I) à (IV) détaillées ci-dessus. A titre indicatif, le disulfure peut être choisi parmi les composés comprenant au moins une chaîne grasse, plus particulièrement au moins une chaîne hydrocarbonée en C₅-C₃₀, linéaire ou ramifiée, saturée ou non, éventuellement substituée par un hétéroatome, éventuellement interrompue par un groupement carboxylique, neutralisé ou non. A titre d'exemple de composés de ce type, on peut citer les dimères de l'acide thioglycolique et ses dérivés du type CH₃-(CH₂)₁₇-S-S-(CH₂)₁₇-CH₃ ou CH₃-(CH₂) -S-S-(CH₂)₁₀-CH₃

S'il est présent, la teneur en ce composé est comprise entre 0,001 et 10 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale est généralement compris entre 3 et 14 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (A) suivante : 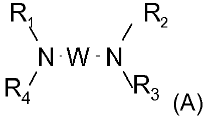 dans laquelle W est un reste propylène éventuellement substitué par un groupement hydroxyle ou un radical alkyle en C₁-C₄ ; R₁, R₂, R₃ et R₄, identiques ou différents, représentent un atome d'hydrogène, un radical alkyle en C₁-C₄ ou hydroxyalkyle en C₁-C₄.

La composition tinctoriale peut se présenter sous des formes diverses, telles que sous forme de liquide, de crème, de gel, ou sous toute autre forme appropriée pour réaliser une teinture des fibres kératiniques, et notamment des cheveux.

Selon un mode de réalisation particulier, le procédé de l'invention comprend un pré-traitement avec un agent réducteur capable de réduire la liaison disulfure. L'agent réducteur est par exemple choisi parmi les thiols par exemple l'acide thioglycolique, la cystéine, l'homocystéine, l'acide thiolactique, les sels de ces thiols, les phosphines, le bisulfite, les sulfites.

Cet agent réducteur peut aussi être choisi parmi les borohydrures et leurs dérivés, comme par exemple les sels du borohydrure, du cyanoborohydrure, du triacétoxyborohydrure, du triméthoxyborohydrure : sels de sodium, lithium, potassium, calcium, ammoniums quaternaires (tetraméthylammonium, tetraéthylammonium, tétra n-butylammonium, benzyltriéthylammonium); le catécholborane.

Ce prétraitement peut être de courte durée, de 0,1 seconde à 30 minutes, de préférence de 0,1 seconde à 5 minutes avec une agent réducteur tel que cité précédemment.

L'application de la composition tinctoriale est généralement effectuée à température ambiante. Elle peut cependant être réalisée à des températures variant de 20 à 180°C.

Selon une variante, l'agent réducteur est ajouté à la composition tinctoriale au moment de l'emploi.

Selon une autre variante, l'application de la composition tinctoriale peut être suivie par une étape courte de réduction de 0,1 seconde à 30 minutes de préférence de 0,1 seconde à 5 minutes, avec un agent réducteur de type thiol ou borohydrure tel que décrit précédemment.

Suivant une autre variante, la composition tinctoriale peut comporter un agent d'oxydation, on parle alors de composition prête à l'emploi.

Habituellement, ladite composition est obtenue en mélangeant la composition selon l'invention avec une composition oxydante avant l'application sur les matières kératiniques à traiter.

L'agent oxydant peut être n'importe quel agent oxydant utilisé de façon classique dans le domaine. Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydo-réductases à 2 électrons telles que les uricases et les oxygénases à 4 électrons comme les laccases. L'utilisation du peroxyde d'hydrogène est particulièrement préférée.

La teneur en agent oxydant est en général comprise entre 1 et 40 % en poids, par rapport au poids de la composition prêt à l'emploi de préférence entre 1 et 20 % en poids par rapport au poids de la composition prêt à l'emploi.

Généralement, la composition oxydante utilisée est une composition aqueuse et peut se trouver sous la forme d'une solution ou encore d'une émulsion.

Habituellement, on mélange la composition tinctoriale exempte d'agent oxydant avec environ 0,5 à 10 équivalents en poids de la composition oxydante.

Notons que le pH de la composition prête à l'emploi est plus particulièrement compris entre 4 et 12, de préférence entre 7 et 11,5.

L'application de la composition tinctoriale peut être suivie d'une étape de post-traitement oxydant, ou d'une étape de post-traitement conditionneur éventuellement associé à une étape de post-traitement oxydant.

L'invention a aussi pour objet un dispositif à plusieurs compartiments ou "kit" de teinture dans lequel un premier compartiment renferme une composition tinctoriale comprenant au moins un colorant disulfure de formules (I), (II), (III) ou (IV) et un deuxième compartiment renferme un agent réducteur capable de réduire la liaison disulfure du colorant.

L'un de ces compartiments peut en outre contenir un ou plusieurs autres colorants de type colorant direct ou colorant d'oxydation à la condition que le colorant disulfure utile dans l'invention et les colorants de type colorant direct ou colorant d'oxydation ne soient pas présents dans le même compartiments du kit.

Elle concerne aussi un dispositif à plusieurs compartiments dans lequel un premier compartiment contient une composition tinctoriale comprenant au moins un colorant disulfure de formules (I), (II) ou (III) ou (IV); un deuxième compartiment contient un agent réducteur capable de réduire la liaison disulfure du colorant ; un troisième compartiment contient un agent oxydant.

Chacun des dispositifs mentionnés ci-dessus peut être équipé d'un moyen permettant de délivrer sur les cheveux le mélange souhaité, par exemple tel que les dispositifs décrits dans le brevet FR2 586 913.

Les exemples qui suivent servent à illustrer l'invention sans toutefois présenter un caractère limitatif.

**EXEMPLES**

**EXEMPLES DE SYNTHESE**

## Revendications

1. Procédé de coloration des fibres kératiniques humaines **caractérisé en ce qu'**on applique sur les fibres, une composition tinctoriale, comprenant dans un milieu cosmétique approprié, au moins un colorant disulfure choisi parmi les colorants de formules (I), (II), (III) ou (IV) suivantes :
A- (X)ₚ - Cₛₐₜ - S-S - Cₛₐₜ - (X)ₚ - A (I) 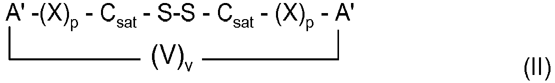 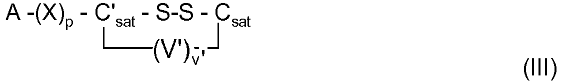
A- (X)ₚ - Cₛₐₜ - S-S - Cₛₐₜ - (X)ₚ - D (IV)
leurs sels, solvates tels que hydrates,
formules dans lesquelles :
• A, et A', identiques ou différents, représentent un radical contenant au moins un chromophore issu de colorants de type cyanines, lesdits chromophores A et/ou A' contiennent un radical cationique porté par ou inclus dans au moins un des chromophores, ledit radical cationique est un ammonium quaternaire ;
• V et V', identiques ou différents représentent un groupe pontant
• v et v', identiques ou différents représentent 0 ou 1 ;
• X, identiques ou non, représentent une chaîne hydrocarbonée en C₁-C₃₀, linéaire ou ramifiée, saturée ou insaturée, éventuellement interrompue et/ ou éventuellement terminée à l'une ou deux de ses extrémités par un ou plusieurs groupes divalents ou leur combinaisons choisis parmi :
- -N(R)-, -N⁺(R)(R)-, -O-, -S-, -CO-, -SO₂- avec R, identiques ou différents, choisi parmi un hydrogène, un radical alkyle en C1-C4, hydroxyalkyle, aminoalkyle
- un radical (hétéro)cyclique aromatique ou non, saturé ou insaturé, condensé ou non comprenant éventuellement un ou plusieurs hétéroatomes identiques ou non, éventuellement substitué ;
• le coefficient p est égal à 0 ou 1 ;
• Cₛₐₜ, C'ₛₐₜ, identiques ou différents représentent une chaîne alkylène en C₁-C₁₈, linéaire ou ramifiée, éventuellement substituée, éventuellement cyclique
• D correspond à un radical choisi parmi les radicaux hydroxy, hydroxyalkyle, alcoxy, carboxyliques, carboxylates, amino, alkylamino, dialkylamino.

2. Procédé selon la revendication 1, **caractérisé en ce que** dans les formules (I), (II), (III) ou (IV), lorsque p est égal à 1, X représente la séquence suivante :
-(T)ₜ-(Y)_{y}-(Z)_{z}-
ladite séquence étant reliée dans les formules (I), (II), (III) ou (IV), comme suit :
- Cₛₐₜ (ou C'ₛₐₜ)-(T)ₜ-(Y)_{y}-(Z)_{z}-(A ou A') ; dans laquelle
**T** représente un ou plusieurs radicaux ou leurs combinaisons choisis parmi -SO₂-, -O-, -S-,-N(R)-, -N+(R)(R) -CO-, R représentant un atome d'hydrogène, un radical alkyle en C₁-C₄, ou hydroxyalkyle en C1-C4;
le coefficient **t** vaut 0 ou 1 ;
**Y** représente :
- un radical choisi parmi -(CH₂)₂-SO₂- ; -CH₂-CHR-CO-NR'- avec R, R', identiques ou non, représentant un atome d'hydrogène, un radical alkyle en C₁-C₄.
- un groupement de formule (a), (a') ou (a") : 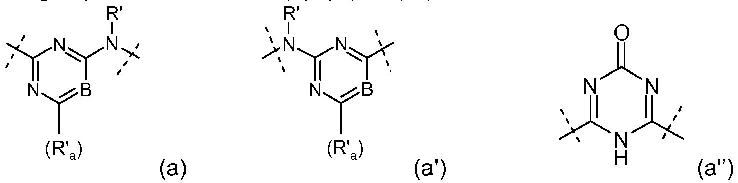 dans laquelle
• B représente -N-, -CRₐ, avec Rₐ représentant un atome d'hydrogène, un atome d'halogène choisi parmi le chlore ou le fluor, un groupement nitro, un groupement pyridinium éventuellement substitué;
• R' a la même définition que précédemment
• R'ₐ représente :
- un atome d'hydrogène
- un atome de chlore ou un atome de fluor
- un groupement pyridinium éventuellement substitué par au moins un groupement R_{c}, R_{c} prouvant être un groupement alkyle en C₁-C₄, un atome d'halogène, un groupement carboxylique -COOM, (avec M représentant un atome d'hydrogène, un métal alcalin, un groupement ammonium ou un groupement ammonium substitué par un ou plusieurs radicaux alkyle, identiques ou non, linéaires ou ramifiés, en C1-C18, éventuellement porteurs d'au moins un hydroxyle) ; un groupement ester-COOR_{d} avec R_{d} représentant un radical alkyle en C₁-C₄ ; un groupement amide -CON(R_{d})₂ avec R_{d} identiques ou non, représentant un atome d'hydrogène ou un radical alkyle en C₁-C₄ ;
- un groupement hydroxyle
- un groupement amino, alkylamino ou dialkylamino, les groupements alkyles identiques ou différents, en C1-C18, étant linéaires ou ramifiés, éventuellement interrompus par un hétéroatome choisi parmi N, O, éventuellement substitués par un ou plusieurs groupements hydroxyles
- un groupement NHNHCOR où R représente un groupement alkyle linéaire ou ramifié en C1-C10
- un groupement de formule (b) suivante : 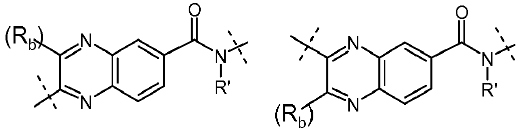 dans laquelle
• R' a la même définition que précédemment
• R_{b} représente
- un atome de chlore
- un groupement amino, alkylamino ou dialkylamino, les groupements alkyles identiques ou différents, en C₁-C₁₈, étant linéaires ou ramifiés, éventuellement interrompus par un hétéroatome choisi parmi N, O, S, éventuellement substitués par un ou plusieurs hydroxyles
- un hétérocycle azoté, saturé ou insaturé, pouvant être substitué
- un groupement arylamino, dans lequel de préférence le radical aryle est en C₆ ;
**y** vaut 0 ou 1 ;
**Z** représente
- - -(CH₂)ₘ- avec m entier compris entre 1 et 8
- - -(CH₂CH₂O)_{q}- ou -(OCH₂CH₂)_{q}- dans lesquelles q est un entier compris entre 1 et 15
- un radical aryle, alkylaryle ou arylalkyle dont le radical alkyle est en C₁-C₄ et le radical aryle est de préférence en C₆, étant éventuellement substitué par au moins un groupement SO₃M avec M représentant un atome d'hydrogène, un métal alcalin ou un groupement ammonium ammonium substitué par un ou plusieurs radicaux alkyle, identiques ou non, linéaires ou ramifiés, en C1-C18 éventuellement porteurs d'au moins un hydroxyle
**z** vaut 0 ou 1.

3. Procédé selon la revendication 1 ou 2, **caractérisé en ce que** Y représente : 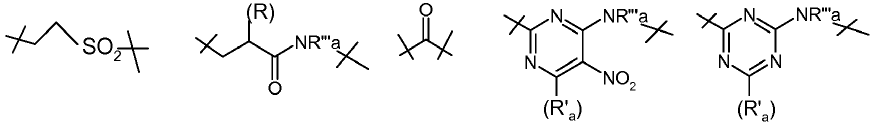 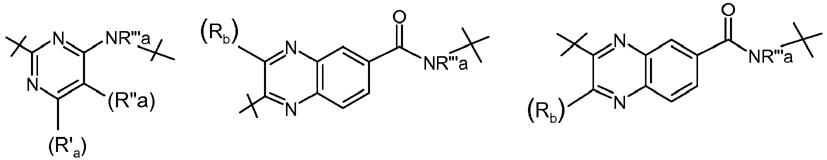 dans lesquels les radicaux R, R'ₐ et R_{b} sont définis comme précédemment ; R"ₐ, a la même définition que R'ₐ, indépendamment les uns des autres ; R'''ₐ représente un atome d'hydrogène ou un radical alkyle.

4. Procédé selon l'une quelconque des revendications précédentes, caractérisé en ce Z représente :

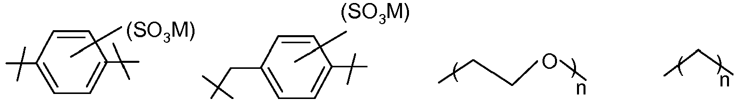

5. Procédé selon l'une quelconque des revendications précédentes, **caractérisé en ce que** le colorant disulfure est tel que v est égal à 0.

6. Procédé selon l'une quelconque des revendications 1 à 5, dans lequel le colorant disulfure est choisi parmi

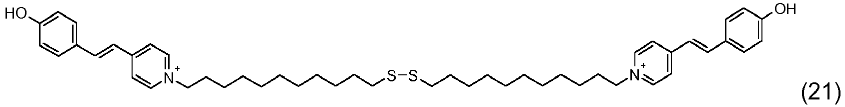

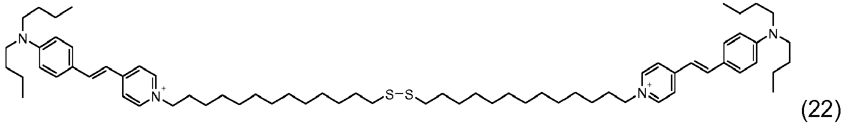

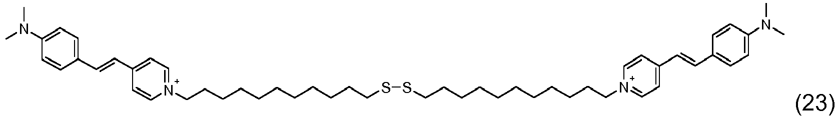

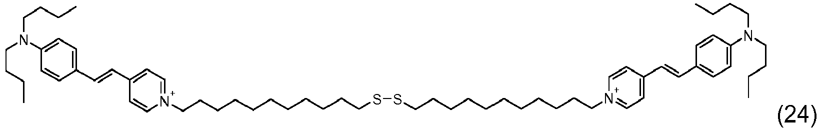

7. Procédé selon l'une quelconque des revendications précédentes dans lequel la composition contient un agent réducteur.

8. Procédé selon l'une quelconque des revendications 1 à 6 comprenant un pré-traitement avec un agent réducteur.

9. Procédé selon l'une quelconque des revendications 1 à 6 comprenant un post-traitement avec un agent réducteur.

10. Procédé selon la revendication 7 à 9 dans lequel l'agent réducteur est choisi parmi les thiols, les phosphines, le bisulfite, les sulfites.

11. Procédé selon la revendication précédente , **caractérisé en ce que** l'agent réducteur est choisi l'acide thioglycolique, la cystéine, l'homocystéine, l'acide thiolactique, les sels de ces thiols.

12. Procédé selon la revendication 7 à 9 dans lequel l'agent réducteur est choisi parmi les borohydrures ou leurs dérivés, comme par exemple les sels du borohydrure, du cyanoborohydrure, du triacétoxyborohydrure, du trimethoxyborohydrure : sels de sodium, lithium, potassium, calcium, ammonium quaternaires comme tétraméthylammonium, tétraéthylammonium, tétra n-butylammonium, benzyltriethylammonium ; le catécholborane.

13. Procédé selon l'une quelconque des revendications 1 à 6, dans lequel la composition comprend un agent oxydant.

14. Procédé selon l'une quelconque des revendications 1 à 6 comprenant un post-traitement avec un agent oxydant.

15. Procédé selon l'une quelconque des revendications 1 à 6 comprenant une étape de post traitement conditionneur éventuellement associé à un post-traitement oxydant.

16. Procédé selon l'une quelconque des revendications 13 ou 14, dans lequel l'agent oxydant est choisi parmi le peroxyde d'hydrogène, le peroxyde d'urée, les bromates de métaux alcalins, les persels tels que les perborates et les persulfates, ainsi que les enzymes.

17. Procédé selon l'une quelconque des revendications précédentes, dans lequel le colorant disulfure est présent en quantité comprise entre 0,001 et 50% en poids, de préférence, entre 0,005 et 20% en poids et encore plus préférentiellement entre 0,01 et 5% en poids, par rapport au poids total de la composition.

18. Procédé selon l'une quelconque des revendications précédentes dans lequel la composition contient au moins un composé disulfure additionnel différent du composé de formule (I), (II), (III) ou (IV).

19. Procédé selon la revendication précédente, **caractérisé en ce que** le composé disulfure autre que les composés de formule (I), (II), (III) ou (IV) est choisi parmi les composés comprenant au moins une chaîne grasse.

20. Procédé selon l'une quelconque des revendications précédentes, dans lequel la composition comprend au moins une base d'oxydation, un coupleur et/ou un colorant direct autre qu'un colorant disulfure.

21. Composition tinctoriale comprenant dans un milieu cosmétique approprié, un colorant disulfure de formules (I), (II), (III) ou (IV) telle que définie précédemment aux revendications 1 à 6.

22. Composition selon la revendication 21 comprenant au moins une base d'oxydation, un coupleur et/ou un colorant direct autre qu'un colorant disulfure.

23. Composition selon l'une quelconque des revendications 21 ou 22 comprenant au moins un agent oxydant.

24. Composition selon l'une quelconque des revendications 21 à 23 comprenant un solvant organique et/ou un agent épaississant.

25. Dispositif à plusieurs compartiments dans lequel un premier compartiment contient une composition tinctoriale comprenant un colorant disulfure tel que défini aux revendications 1 à 6 et un deuxième compartiment contient un agent réducteur capable de réduire une liaison disulfure.

26. Dispositif selon la revendication 25 comprenant un troisième compartiment qui contient un agent oxydant.

27. Utilisation des colorants disulfure tels que définis aux revendications 1 à 6 pour la teinture de fibres kératiniques humaines, en particulier les cheveux.

28. Utilisation selon la revendication 27 pour améliorer la ténacité de la coloration.

29. Colorant disulfure cationique comprenant au moins un radical ammonium quaternaire, de formules (I), (II), (III) ou (IV) suivantes, :
A- (X)ₚ - Cₛₐₜ - S-S - Cₛₐₜ - (X)ₚ - A (I) 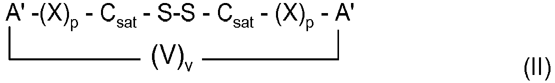 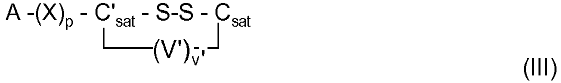
A- (X)ₚ - Cₛₐₜ - S-S - Cₛₐₜ - (X)ₚ - D (IV)
leurs sels, solvates tels que hydrates,
formules dans lesquelles :
• A, et A', identiques ou différents, représentent un radical contenant au moins un chromophore issu de colorants de type cyanines, lesdits chromophores A et/ou A' contiennent un radical cationique porté par ou inclus dans au moins un des chromophores, ledit radical cationique est un ammonium quaternaire ;
• V et V', identiques ou différents représentent un groupe pontant ;
• v et v', identiques ou différents représentent 0 ou 1 ;
• X représente la séquence suivante :
-(T)ₜ-(Y)_{y}-(Z)_{z}-
ladite séquence étant reliée dans les formules (I), (II), (III) ou (IV), comme suit :
- Cₛₐₜ (ou C'ₛₐₜ)-(T)ₜ-(Y)_{y}-(Z)_{z}-(A ou A') ; dans laquelle
**T** représente un ou plusieurs radicaux ou leurs combinaisons choisis parmi -SO₂-, -O-,-S-, -N(R)-, -N+(R)(R) -CO-, R représentant un atome d'hydrogène, un radical alkyle en C₁-C₄, ou hydroxyalkyle en C₁-C₄;
le coefficient **t** vaut 0 ou 1 ;
**Y** représente :
- un radical choisi parmi -(CH₂)₂-SO₂- ; -CH₂-CHR-CO-NR'- avec R, R', identiques ou non, représentant un atome d'hydrogène, un radical alkyle en C₁-C₄.
- un groupement de formule (a), (a') ou (a") : 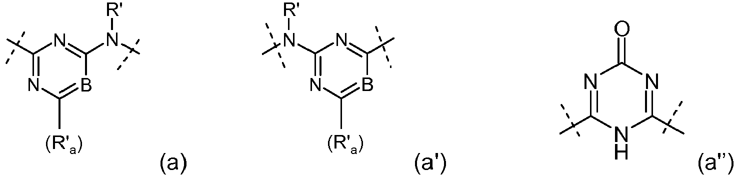 dans laquelle
• B représente -N-, -CRₐ, avec Rₐ représentant un atome d'hydrogène, un atome d'halogène choisi parmi le chlore ou le fluor, un groupement nitro, un groupement pyridinium éventuellement substitué;
• R' a la même définition que précédemment
• R'ₐ représente :
- un atome d'hydrogène
- un atome de chlore ou un atome de fluor
- un groupement pyridinium éventuellement substitué par au moins un groupement R_{c}, R_{c} prouvant être un groupement alkyle en C₁-C₄, un atome d'halogène, un groupement carboxylique -COOM, (avec M représentant un atome d'hydrogène, un métal alcalin, un groupement ammonium ou un groupement ammonium substitué par un ou plusieurs radicaux alkyle, identiques ou non, linéaires ou ramifiés, en C₁-C₁₈, éventuellement porteurs d'au moins un hydroxyle) ; un groupement ester-COOR_{d} avec R_{d} représentant un radical alkyle en C₁-C₄ ; un groupement amide -CON(R_{d})₂ avec R_{d} identiques ou non, représentant un atome d'hydrogène ou un radical alkyle en C₁-C₄ ;
- un groupement hydroxyle
- un groupement amino, alkylamino ou dialkylamino, les groupements alkyles identiques ou différents, en C₁-C₁₈, étant linéaires ou ramifiés, éventuellement interrompus par un hétéroatome choisi parmi N, O, éventuellement substitués par un ou plusieurs groupements hydroxyles
- un groupement NHNHCOR où R représente un groupement alkyle linéaire ou ramifié en C₁-C₁₀
- un groupement de formule (b) suivante : 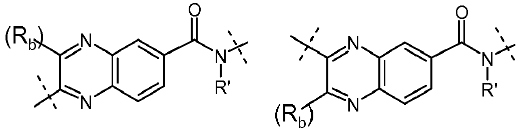 dans laquelle
• R' a la même définition que précédemment
• R_{b} représente
- un atome de chlore
- un groupement amino, alkylamino ou dialkylamino, les groupements alkyles identiques ou différents, en C₁-C₁₈, étant linéaires ou ramifiés, éventuellement interrompus par un hétéroatome choisi parmi N, O, S, éventuellement substitués par un ou plusieurs hydroxyles
- un hétérocycle azoté, saturé ou insaturé, pouvant être substitué
• un groupement arylamino, dans lequel de préférence le radical aryle est en C₆ ;
• le coefficient p est égal à 1 ;
• **y** vaut 0 ou 1 ;
• **Z** représente :
- - -(CH₂)ₘ- avec m entier compris entre 1 et 8
- - -(CH₂CH₂O)_{q}- ou -(OCH₂CH₂)_{q}- dans lesquelles q est un entier compris entre 1 et 15
- un radical aryle, alkylaryle ou arylalkyle dont le radical alkyle est en C₁-C₄ et le radical aryle est de préférence en C₆, étant éventuellement substitué par au moins un groupement SO₃M avec M représentant un atome d'hydrogène, un métal alcalin ou un groupement ammonium ammonium substitué par un ou plusieurs radicaux alkyle, identiques ou non, linéaires ou ramifiés, en C1-C18 éventuellement porteurs d'au moins un hydroxyle
• **z** vaut 0 ou 1.
• Cₛₐₜ, C'ₛₐₜ, identiques ou différents représentent une chaîne alkylène en C₁-C₁₈, linéaire ou ramifiée, éventuellement substituée, éventuellement cyclique ;
• D correspond à un radical choisi parmi les radicaux hydroxy, hydroxyalkyle, alcoxy, carboxyliques, carboxylates, amino, alkylamino, dialkylamino ;
étant entendu que le composé de formule (I) ne peut pas représenter les composés suivants :

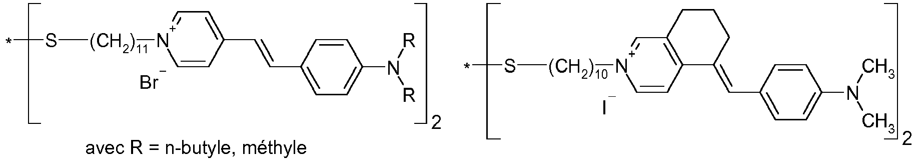

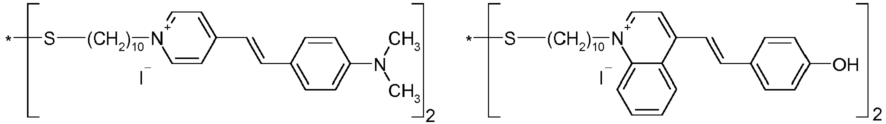

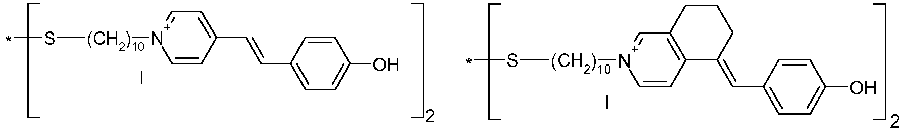

## Patentansprüche

1. Verfahren zum Färben von menschlichen Keratinfasern, **dadurch gekennzeichnet, dass** man auf die Fasern eine Färbezusammensetzung aufbringt, die in einem kosmetisch geeigneten Medium mindestens einen Disulfid-Farbstoff umfasst, der aus Farbstoffen der folgenden Formeln (I), (II), (III) oder (IV):
A- (X)ₚ - Cₛₐₜ - S-S - Cₛₐₜ - (X)ₚ - A (I) 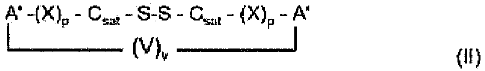 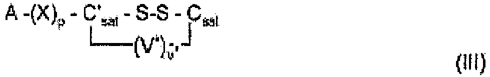
A- (X)ₚ - Cₛₐₜ - S-S - Cₛₐₜ - (X)ₚ - D (IV),
deren Salzen und Solvaten wie Hydraten ausgewählt wird,
wobei in den Formeln:
• A und A' gleich oder verschieden sind und für einen Rest, der mindestens einen Chromophor, der sich von Farbstoffen vom Cyanin-Typ ableitet, enthält, stehen, wobei die Chromophore A und/oder A' einen von mindestens einem der Chromophore getragenen oder in mindestens einem der Chromophore enthaltenen kationischen Rest enthalten, wobei es sich bei dem kationischen Rest um ein quaternäres Ammonium handelt;
• V und V' gleich oder verschieden sind und für eine Brückengruppe stehen;
• v und v' gleich oder verschieden sind und für 0 oder 1 stehen;
• die Variablen X gleich oder verschieden sind und für eine gesättigte oder ungesättigte, lineare oder verzweigte C₁-C₃₀-Kohlenwasserstoffkette, die gegebenenfalls durch eine oder mehrere zweiwertige Gruppen oder Kombinationen davon, die aus:
- -N(R)-, -N⁺ (R) (R)-, -O-, -S-, -CO- und -SO₂-, wobei R gleich oder verschieden ist und aus einem Wasserstoff und einem C₁-C₄-Alkyl-, Hydroxyalkyl- und Aminoalkylrest ausgewählt ist;
- einem kondensierten oder nichtkondensierten, gesättigten oder ungesättigten, aromatischen oder nichtaromatischen (hetero)cyclischen Rest, der gegebenenfalls ein oder mehrere gleiche oder verschiedene Heteroatome enthält und gegebenenfalls substituiert ist,
ausgewählt sind, unterbrochen und/oder an einem oder beiden ihrer Enden durch diese Gruppen oder Kombinationen davon terminiert ist, stehen;
• der Koeffizient p gleich 0 oder 1 ist;
• Cₛₐₜ und C'ₛₐₜ gleich oder verschieden sind und für eine gegebenenfalls cyclische, gegebenenfalls substituierte, lineare oder verzweigte C₁-C₁₈-Alkylenkette stehen;
• D einem aus Hydroxy-, Hydroxyalkyl-, Alkoxy-, Carboxyl-, Carboxylat-, Amino-, Alkylamino- und Dialkylaminoresten ausgewählten Rest entspricht.

2. Verfahren nach Anspruch 1, **dadurch gekennzeichnet, dass** in den Formeln (I), (II), (III) oder (IV) dann, wenn p gleich 1 ist, X für die folgende Sequenz steht:
-(T)ₜ-(Y)_{y}-(Z)_{z}-,
wobei die Sequenz in den Formeln (I), (II), (III) oder (IV) folgendermaßen gebunden ist:
- Cₛₐₜ(bzw. C'ₛₐₜ)-(T)ₜ-(Y)_{y}-(Z)_{z}-A (bzw. A'); worin:
**T** für einen oder mehrere Reste oder Kombinationen davon, die aus -SO₂-, -O-, -S-, -N(R)-, -N⁺ (R) (R')- und -CO- ausgewählt sind, wobei R und R' gleich oder verschieden sind und für ein Wasserstoffatom oder einen C₁-C₄-Alkyl- oder C₁-C₄-Hydroxyalkylrest stehen, steht;
der Koeffizient **t** gleich 0 oder 1 ist;
**Y** für:
- einen Rest, der aus - (CH₂)₂-SO₂- und -CH₂-CHR-CO-NR'- ausgewählt ist, wobei R und R' gleich oder verschieden sind und für ein Wasserstoffatom oder einen C₁-C₄-Alkylrest stehen;
- eine Gruppe der Formel (a), (a') oder (a"): 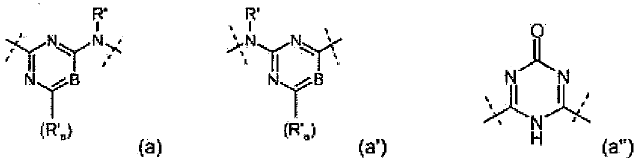 worin:
• B für -N- oder -CRₐ- steht, wobei Rₐ für ein Wasserstoffatom, ein Halogenatom, das aus Chlor oder Fluor ausgewählt ist, eine Nitrogruppe, oder eine gegebenenfalls substituierte Pyridiniumgruppe steht;
• R' die gleiche Definition wie oben hat;
• R'ₐ für:
- ein Wasserstoffatom,
- ein Chloratom oder ein Fluoratom,
- eine Pyridiniumgruppe, die gegebenenfalls durch mindestens eine Gruppe R_{c} substituiert ist, wobei R_{c} für eine C₁-C₄-Alkylgruppe, ein Halogenatom, eine Carboxylgruppe -COOM (wobei M für ein Wasserstoffatom, ein Alkalimetall, eine Ammoniumgruppe oder eine durch einen oder mehrere gleiche oder verschiedene lineare oder verzweigte C₁-C₁₈-Alkylreste, die gegebenenfalls mindestens ein Hydroxyl tragen, substituierte Ammoniumgruppe steht); eine Estergruppe -COOR_{d}, wobei R_{d} für einen C₁-C₄-Alkylrest steht; oder eine Amidgruppe -CON(R_{d})₂, wobei die Rₐ-Gruppen gleich oder verschieden sind und für ein Wasserstoffatom oder einen C₁-C₄-Alkylrest stehen, stehen kann;
- eine Hydroxylgruppe,
- eine Amino-, Alkylamino- oder Dialkylaminogruppe, wobei die gleichen oder verschiedenen linearen oder verzweigten C₁-C₁₈-Alkylgruppen gegebenenfalls durch ein Heteroatom, das aus N und O ausgewählt ist, unterbrochen und gegebenenfalls durch eine oder mehrere Hydroxylgruppen substituiert sind,
- eine NHNHCOR-Gruppe, wobei R für eine lineare oder verzweigte C₁-C₁₀-Alkylgruppe steht,
steht,
- eine Gruppe der folgenden Formel (b): 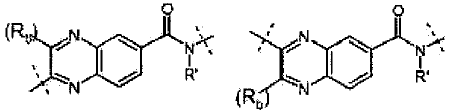 worin
• R' die gleiche Definition wie oben hat;
• R_{b} für:
- ein Chloratom,
- eine Amino-, Alkylamino- oder Dialkylaminogruppe, wobei die gleichen oder verschiedenen linearen oder verzweigten C₁-C₁₈-Alkylgruppen gegebenenfalls durch ein Heteroatom, das aus N, O und S ausgewählt ist, unterbrochen und gegebenenfalls durch eine oder mehrere Hydroxylgruppen substituiert sind,
- einen gesättigten oder ungesättigten stickstoffhaltigen Heterocyclus, der substituiert sein kann,
- eine Arylaminogruppe, wobei der Arylrest vorzugsweise C₆ ist, steht,
steht;
**y** gleich 0 oder 1 ist;
**Z** für:
- -(CH₂)ₘ-, wobei m für eine ganze Zahl zwischen 1 und 8 steht,
- - (CH₂CH₂O)_{q}- oder - (OCH₂CH₂)_{q}- , worin q für eine ganze zahl zwischen 1 und 15 steht,
- einen Aryl-, Alkylaryl- oder Arylalkylrest, dessen Alkylrest C₁-C₄ ist und dessen Arylrest vorzugsweise C₆ ist und der gegebenenfalls durch mindestens eine SO₃M-Gruppe substituiert ist, wobei M für ein Wasserstoffatom, ein Alkalimetall oder eine durch einen oder mehrere gleiche oder verschiedene lineare oder verzweigte C₁-C₁₈-Alkylreste, die gegebenenfalls mindestens ein Hydroxyl tragen, substituierte Ammoniumammoniumgruppe steht,
steht,
**z** für 0 oder 1 steht.

3. Verfahren nach Anspruch 1 oder 2, **dadurch gekennzeichnet, dass** Y für: 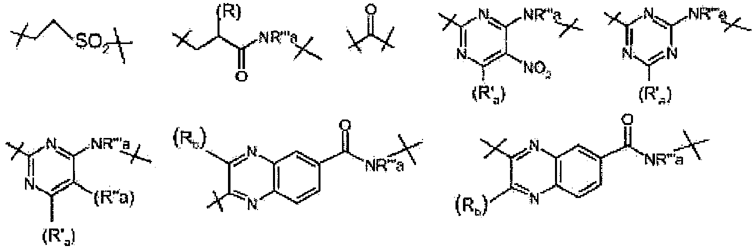 steht, wobei die Reste R, R'ₐ und R_{b} wie oben definiert sind; R"ₐ unabhängig voneinander die gleiche Definition wie R'ₐ hat und R'''ₐ für ein Wasserstoffatom oder einen Alkylrest steht.

4. Verfahren nach einem der vorhergehenden Ansprüche, **dadurch gekennzeichnet, dass** Z für: 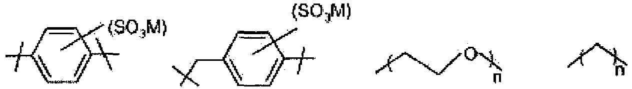 steht.

5. Verfahren nach einem der vorhergehenden Ansprüche, **dadurch gekennzeichnet, dass** der Disulfid-Farbstoff so beschaffen ist, dass v gleich 0 ist.

6. Verfahren nach einem der Ansprüche 1 bis 5, bei dem der Disulfid-Farbstoff aus 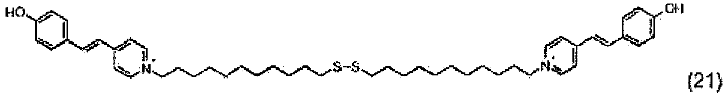 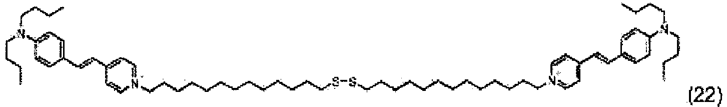 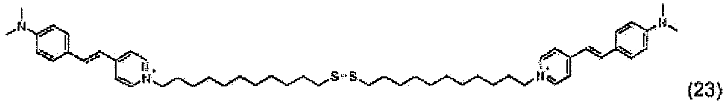 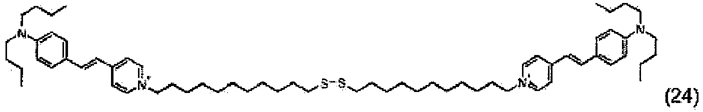 ausgewählt wird.

7. Verfahren nach einem der vorhergehenden Ansprüche, bei dem die Zusammensetzung ein Reduktionsmittel enthält.

8. Verfahren nach einem der Ansprüche 1 bis 6, das eine Vorbehandlung mit einem Reduktionsmittel umfasst.

9. Verfahren nach einem der Ansprüche 1 bis 6, das eine Nachbehandlung mit einem Reduktionsmittel umfasst.

10. Verfahren nach Anspruch 7 bis 9, bei dem das Reduktionsmittel aus Thiolen, Phosphinen, Bisulfit und Sulfiten ausgewählt wird.

11. Verfahren nach dem vorhergehenden Anspruch, **dadurch gekennzeichnet, dass** das Reduktionsmittel aus Thioglykolsäure, Cystein, Homocystein, Thiomilchsäure und Salzen dieser Thiole ausgewählt wird.

12. Verfahren nach Anspruch 7 bis 9, bei dem das Reduktionsmittel aus Borhydriden oder Derivaten davon, wie beispielsweise Borhydrid-, Cyanoborhydrid-, Triacetoxyborhydrid- und Trimethoxyborhydridsalzen: Natriumsalzen, Lithiumsalzen, Kaliumsalzen, Calciumsalzen und quaternären Ammoniumsalzen wie Tetramethylammonium-, Tetraethylammonium-, Tetra-n-butylammonium- und Benzyltriethylammöniumsalzen und Catecholboran, ausgewählt wird.

13. Verfahren nach einem der Ansprüche 1 bis 6, bei dem die Zusammensetzung ein Oxidationsmittel umfasst.

14. Verfahren nach einem der Ansprüche 1 bis 6, das eine Nachbehandlung mit einem Oxidationsmittel umfasst.

15. Verfahren nach einem der Ansprüche 1 bis 6, das einen Schritt der konditionierenden Nachbehandlung, gegebenenfalls in Kombination mit einer oxidierenden Nachbehandlung, umfasst.

16. Verfahren nach einem der Ansprüche 13 oder 14, bei dem das Oxidationsmittel aus Wasserstoffperoxid, Harnstoffperoxid, Alkalimetallbromaten, Persalzen wie Perboraten und Persulfaten sowie Enzymen ausgewählt ist.

17. Verfahren nach einem der vorhergehenden Ansprüche, bei dem der Disulfid-Farbstoff in einer Menge zwischen 0,001 und 50 Gew.-%, vorzugsweise zwischen 0,005 und 20 Gew.-% und noch weiter bevorzugt zwischen 0,01 und 5 Gew.-%, bezogen auf das Gesamtgewicht der Zusammensetzung, vorliegt.

18. Verfahren nach einem der vorhergehenden Ansprüche, bei dem die Zusammensetzung mindestens eine zusätzliche Disulfid-Verbindung, die von der Verbindung der Formel (I), (II), (III) oder (IV) verschieden ist, enthält.

19. Verfahren nach dem vorhergehenden Anspruch, **dadurch gekennzeichnet, dass** die Disulfid-Verbindung, die von der Verbindung der Formel (I), (II), (III) oder (IV) verschieden ist, aus Verbindungen mit mindestens einer Fettkette ausgewählt wird.

20. Verfahren nach einem der vorhergehenden Ansprüche, bei dem die Zusammensetzung mindestens eine Oxidationsbase, einen Kuppler und/oder einen Direktfarbstoff, der von einem Disulfid-Farbstoff verschieden ist, umfasst.

21. Färbezusammensetzung, die die in einem kosmetisch geeigneten Medium einen Disulfid-Farbstoff der Formeln (I), (II), (III) oder (IV) gemäß obiger Definition in den Ansprüchen 1 bis 6 umfasst.

22. Zusammensetzung nach Anspruch 21, die mindestens eine Oxidationsbase, einer Kuppler und/oder einen Direktfarbstoff, der von einem Disulfid-Farbstoff verschieden ist, umfasst.

23. Zusammensetzung nach einem der Ansprüche 21 oder 22, die mindestens ein Oxidationsmittel umfasst.

24. Zusammensetzung nach einem der Ansprüche 21 bis 23, die ein organisches Lösungsmittel und/oder ein Verdickungsmittel umfasst.

25. Vorrichtung mit mehreren Kompartimenten, in der ein erstes Kompartiment eine Färbezusammensetzung, die einen Disulfid-Farbstoff gemäß den Ansprüchen 1 bis 6 umfasst, enthält und ein zweites Kompartiment ein Reduktionsmittel, das eine Disulfid-Bindung reduzieren kann, enthält.

26. Vorrichtung nach Anspruch 25 mit einem dritten Kompartiment, das ein Oxidationsmittel enthält.

27. Verwendung der Disulfid-Farbstoffe gemäß den Ansprüchen 1 bis 6 zum Färben von menschlichen Keratinfasern, insbesondere dem Haar.

28. Verwendung nach Anspruch 27 zur Verbesserung der Echtheit der Färbung.

29. Kationischer Disulfid-Farbstoff mit mindestens einem quaternären Ammoniumrest der folgenden Formeln (I), (II), (III) oder (IV):
A- (X)ₚ - Cₛₐₜ - S-S - Cₛₐₜ - (X)ₚ - A (I) 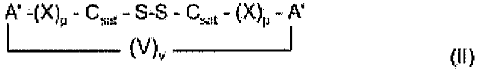 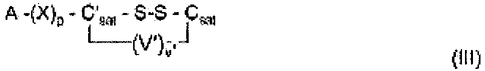
A- (X)ₚ - Cₛₐₜ - S-S - Cₛₐₜ - (X)ₚ - D (IV),
deren Salze und Solvate wie Hydrate,
wobei in den Formeln:
• A und A' gleich oder verschieden sind und für einen Rest, der mindestens einen Chromophor, der sich von Farbstoffen vom Cyanin-Typ ableitet, enthält, stehen, wobei die Chromophore A und/oder A' einen von mindestens einem der Chromophore getragenen oder in mindestens einem der Chromophore enthaltenen kationischen Rest enthalten, wobei es sich bei dem kationischen Rest um ein quaternäres Ammonium handelt;
• V und V' gleich oder verschieden sind und für eine Brückengruppe stehen;
• v und v' gleich oder verschieden sind und für 0 oder 1 stehen;
• X für die folgende Sequenz steht:
-(T)ₜ-(Y)_{y}-(Z)_{z}-,
wobei die Sequenz in den Formeln (I), (II), (III) oder (IV) folgendermaßen gebunden ist:
- Cₛₐₜ(bzw. C'ₛₐₜ) - (T)ₜ-(Y)_{y}-(Z)_{z}-A (bzw. A'); worin:
**T** für einen oder mehrere Reste oder Kombinationen davon, die aus -SO₂-, -O-, -S-, -N(R)-, -N⁺(R)(R')- und -CO- ausgewählt sind, wobei R und R' gleich oder verschieden sind und für ein Wasserstoffatom oder einen C₁-C₄-Alkyl- oder C₁-C₄-Hydroxyalkylrest stehen, steht;
der Koeffizient **t** gleich 0 oder 1 ist;
**Y** für:
- einen Rest, der aus - (CH₂)₂-SO₂- und -CH₂-CHR-CO-NR¹- ausgewählt ist, wobei R und R' gleich oder verschieden sind und für ein Wasserstoffatom oder einen C₁-C₄-Alkylrest stehen;
- eine Gruppe der Formel (a), (a') oder (a"): 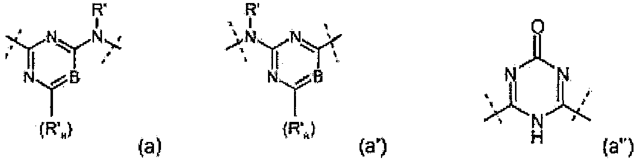 worin:
• B für -N- oder -CRₐ- steht, wobei Rₐ für ein Wasserstoffatom, ein Halogenatom, das aus Chlor oder Fluor ausgewählt ist, eine Nitrogruppe, oder eine gegebenenfalls substituierte Pyridiniumgruppe steht;
• R' die gleiche Definition wie oben hat;
• R'ₐ für:
- ein Wasserstoffatom,
- ein Chloratom oder ein Fluoratom,
- eine Pyridiniumgruppe, die gegebenenfalls durch mindestens eine Gruppe R_{c} substituiert ist, wobei R_{c} für eine C₁-C₄-Alkylgruppe, ein Halogenatom, eine Carboxylgruppe -COOM (wobei M für ein Wasserstoffatom, ein Alkalimetall, eine Ammoniumgruppe oder eine durch einen oder mehrere gleiche oder verschiedene lineare oder verzweigte C₁-C₁₈-Alkylreste, die gegebenenfalls mindestens ein Hydroxyl tragen, substituierte Ammoniumgruppe steht); eine Estergruppe -COOR_{d}, wobei R_{d} für einen C₁-C₄-Alkylrest steht; oder eine Amidgruppe -CON(R_{d})₂, wobei die R_{d}-Gruppen gleich oder verschieden sind und für ein Wasserstoffatom oder einen C₁-C₄-Alkylrest stehen, stehen kann;
- eine Hydroxylgruppe,
- eine Amino-, Alkylamino- oder Dialkylaminogruppe, wobei die gleichen oder verschiedenen linearen oder verzweigten C₁-C₁₈-Alkylgruppen gegebenenfalls durch ein Heteroatom, das aus N und 0 ausgewählt ist, unterbrochen und gegebenenfalls durch eine oder mehrere Hydroxylgruppen substituiert sind,
- eine NHNHCOR-Gruppe, wobei R für eine lineare oder verzweigte C₁-C₁₀-Alkylgruppe steht,
steht,
- eine Gruppe der folgenden Formel (b): 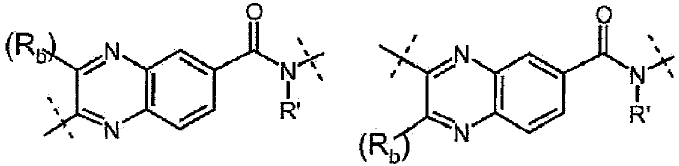 worin
• R' die gleiche Definition wie oben hat;
• R_{b} für:
- ein Chloratom,
- eine Amino-, Alkylamino- oder Dialkylaminogruppe, wobei die gleichen oder verschiedenen linearen oder verzweigten C₁-C₁₈-Alkylgruppen gegebenenfalls durch ein Heteroatom, das aus N, O und S ausgewählt ist, unterbrochen und gegebenenfalls durch eine oder mehrere Hydroxylgruppen substituiert sind,
- einen gesättigten oder ungesättigten stickstoffhaltigen Heterocyclus, der substituiert sein kann,
- eine Arylaminogruppe, wobei der Arylrest vorzugsweise C₆ ist, steht,
steht;
**y** gleich 0 oder 1 ist;
**Z** für:
- - (CH₂)ₘ-, wobei m für eine ganze Zahl zwischen 1 und 8 steht,
- - (CH₂CH₂O)_{q}- oder -(OCH₂CH₂)_{q}-, worin q für eine ganze Zahl zwischen 1 und 15 steht,
- einen Aryl-, Alkylaryl- oder Arylalkylrest, dessen Alkylrest C₁-C₄ ist und dessen Arylrest vorzugsweise C₆ ist und der gegebenenfalls durch mindestens eine SO₃M-Gruppe substituiert ist, wobei M für ein Wasserstoffatom, ein Alkalimetall oder eine durch einen oder mehrere gleiche oder verschiedene lineare oder verzweigte C₁-C₁₈-Alkylreste, die gegebenenfalls mindestens ein Hydroxyl tragen, substituierte Ammoniumammoniumgruppe steht,
steht, **z** für 0 oder 1 steht
• Cₛₐₜ und C'ₛₐₜ gleich oder verschieden sind und für eine gegebenenfalls cyclische, gegebenenfalls substituierte, lineare oder verzweigte C₁-C₁₈-Alkylenkette stehen;
• D einem aus Hydroxy-, Hydroxyalkyl-, Alkoxy-, Carboxyl-, Carboxylat-, Amino-, Alkylamino- und Dialkylaminoresten ausgewählten Rest entspricht;
mit der Maßgabe, dass die Verbindungen der Formel (I) nicht für die folgenden Verbindungen stehen können: 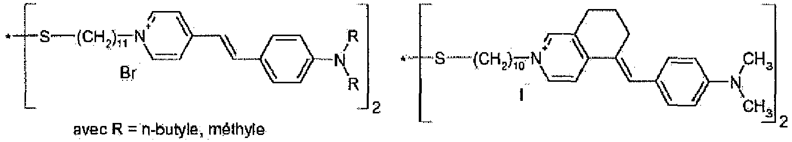 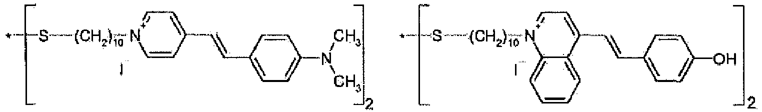 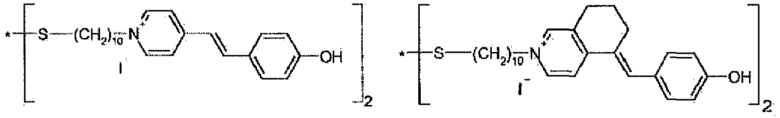 [avec R = n-butyle, méthyle -> mit R = n-Butyl, Methyl]

## Claims

1. Method for dyeing human keratin fibres, **characterized in that** one applies to the fibres a dyeing composition comprising, in an appropriate cosmetic medium, at least one disulfide dye chosen from the dyes of the following formulae (I), (II), (III) or (IV) :
A- (X)ₚ - Cₛₐₜ - S-S - Cₛₐₜ - (X)ₚ - A (I) 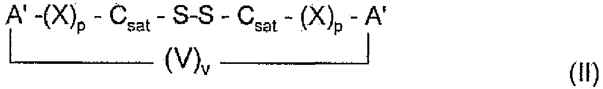 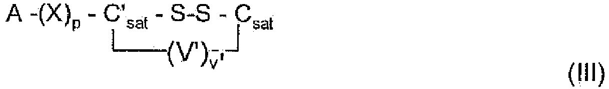
A- (X)ₚ - Cₛₐₜ - S-S - Cₛₐₜ - (X)ₚ - D (IV)
their salts or solvates such as hydrates,
in which formulae:
• A and A', which are identical or different, represent a radical containing at least one chromophore derived from cyanine-type dyes, said chromophores A and/or A' contain a cationic radical borne by or included in at least one of the chromophores; said cationic radical B is quaternary ammonium ;
• V and V', which are identical or different, represent a bridging group;
• v and v', which are identical or different, represent 0 or 1;
• X, which are identical or different, represent a saturated or unsaturated, linear or branched C₁-C₃₀ hydrocarbon chain optionally interrupted and/or optionally terminated at one or both of its ends by one or more divalent groups or combinations thereof, chosen from:
- -N(R)-, -N⁺(R)(R)-, -O-, -S-, -CO-, -SO₂- with R, which are identical or different, being chosen from hydrogen, a C₁-C₄ alkyl, hydroxyalkyl or aminoalkyl radical;
- an optionally substituted, saturated or unsaturated, fused or nonfused, aromatic or nonaromatic (hetero) cyclic radical optionally comprising one or more identical or different heteroatoms;
• the coefficient p is equal to 0 or 1;
• Cₛₐₜ, C'ₛₐₜ, which are identical or different, represent an optionally substituted, optionally cyclic, linear or branched C₁-C₁₈ alkylene chain;
• D corresponds to a radical chosen from hydroxyl, hydroxyalkyl, alkoxy, carboxyl, carboxylate, amino, alkylamino and dialkylamino radicals.

2. Method according to Claim 1, **characterized in that** in formulae (I), (II), (III) or (IV), when p is equal to 1, X represents the following sequence:
- (T)ₜ-(Y)_{y}-(Z)_{z}-
the said sequence being linked in formulae (I), (II), (III) or (IV) as follows:
- Cₛₐₜ(or C'ₛₐₜ)-(T)ₜ-(Y)_{y}-(Z)_{z}-(A or A'); in which
**T** represents one or more radicals or combinations thereof, chosen from -SO₂-, -O-, -S-, -N(R)-, -N+ (R) (R) -CO-, R representing a hydrogen atom, a C₁-C₄ alkyl radical or a C₁-C₄ hydroxyalkyl radical;
the coefficient **t** is equal to 0 or 1;
**Y** represents:
- a radical chosen from -(CH₂)₂-SO₂-;
- CH₂-CHR-CO-NR'- with R, R', which are identical or different, representing a hydrogen atom, a C₁-C₄ alkyl radical.
- a group of formula (a), (a') or (a"): 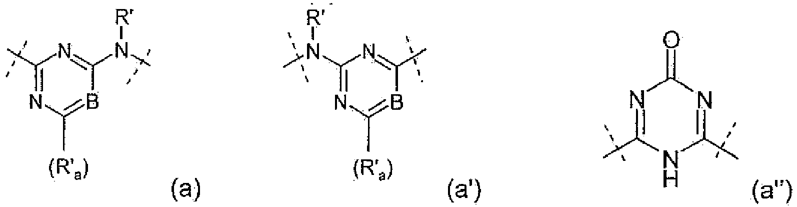 in which
• B represents -N-, -CRₐ, with Rₐ representing a hydrogen atom, a halogen atom chosen from chlorine or fluorine, a nitro group, a pyridinium group which is optionally substituted;
• R' has the same definition as above
• R'ₐ represents:
- a hydrogen atom
- a chlorine atom or a fluorine atom
- a pyridinium group which is optionally substituted with at least one group R_{c}, it being possible for R_{c} to be a C₁-C₄ alkyl group, a halogen atom, a carboxyl group -COOM (with M representing a hydrogen atom, an alkali metal, an ammonium group or an ammonium group substituted with one or more linear or branched, identical or different C₁-C₁₈ alkyl radicals, optionally bearing at least one hydroxyl); an ester group -COOR_{d} with R_{d} representing a C₁-C₄ alkyl radical; an amide group -CON (R_{d})₂ with R_{d}, which are identical or different, representing a hydrogen atom or a C₁-C₄ alkyl radical;
- a hydroxyl group
- an amino, alkylamino or dialkylamino group, the identical or different C₁-C₁₈ alkyl groups being linear or branched, optionally interrupted by a heteroatom chosen from N, 0, which are optionally substituted with one or more hydroxyl groups
- a group NHNHCOR where R represents a linear or branched C₁-C₁₀ alkyl group
- a group of the following formula (b): 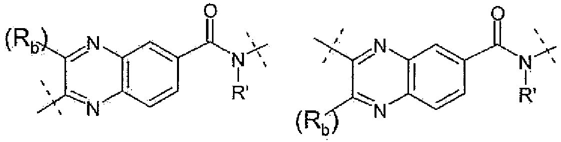 in which
• R' has the same definition as above
• R_{b} represents
- a chlorine atom
- an amino, alkylamino or dialkylamino group, the identical or different C₁-C₁₈ alkyl groups being linear or branched, optionally interrupted by a heteroatom chosen from N, O, S, which are optionally substituted with one or more hydroxyls
- a saturated or unsaturated nitrogen-containing heterocycle which may be substituted
- an arylamino group in which the aryl radical is preferably C₆;
**y** is equal to 0 or 1;
**z** represents
- -(CH₂)ₘ- with m integer between 1 and 8
- - (CH₂CH₂O)_{q}- or - (OCH₂CH₂)_{q}- in which q is an integer between 1 and 15
- an aryl, alkylaryl or arylalkyl radical whose alkyl radical is C₁-C₄ and the aryl radical is preferably C₆, being optionally substituted with at least one group SO₃M with M representing a hydrogen atom, an alkali metal or an ammonium ammonium group substituted with one or more identical or different, linear or branched C₁-C₁₈ alkyl radicals optionally bearing at least one hydroxyl
**z** is equal to 0 or 1.

3. Method according to Claim 1 or 2, **characterized in that** Y represents: 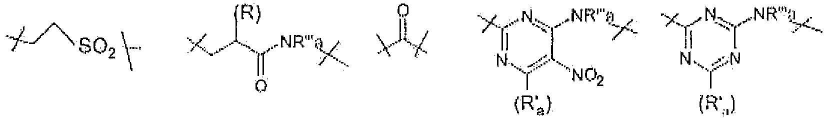 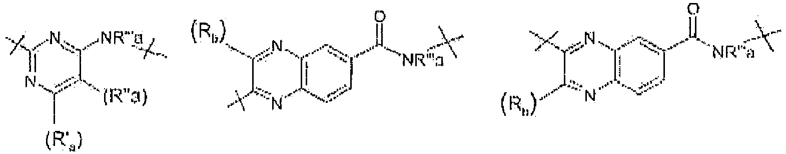 in which the radicals R, R'ₐ and R_{b} are as defined above; R"ₐ has the same definition as R'ₐ, independently of each other; R"ₐ represents a hydrogen atom or an alkyl radical.

4. Method according to any one of the preceding claims, **characterized in that** Z represents:

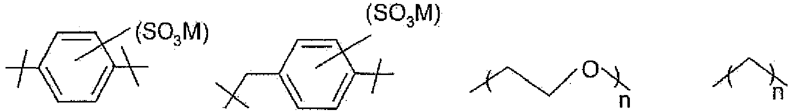

5. Method according to any one of the preceding claims, **characterized in that** the disulfide dye is such that v is equal to 0.

6. Method according to any one of Claims 1 to 5, in which the disulfide dye is chosen from

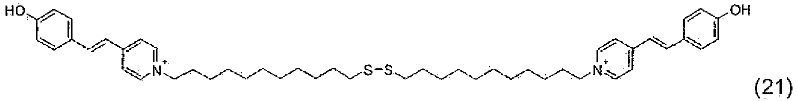

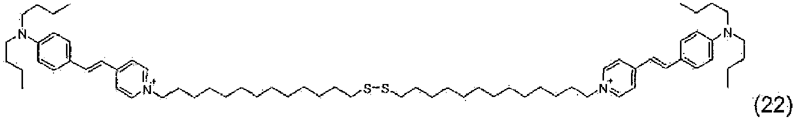

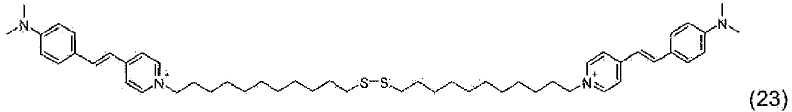

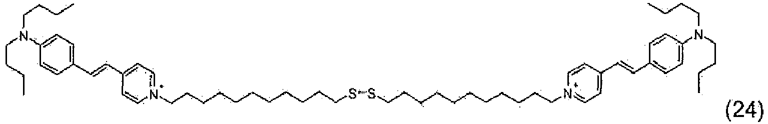

7. Method according to any one of the preceding claims, in which the composition contains a reducing agent.

8. Method according to any one of Claims 1 to 6, comprising a pretreatment with a reducing agent.

9. Method according to any one of Claims 1 to 6, comprising a post-treatment with a reducing agent.

10. Method according to Claims 7 to 9, in which the reducing agent is chosen from thiols, phosphines, bisulfite and sulphites.

11. Method according to the preceding claim, **characterized in that** the reducing agent is chosen from thioglycolic acid, cysteine, homocysteine, thiolactic acid and the salts of these thiols.

12. Method according to Claims 7 to 9, in which the reducing agent is chosen from borohydrides or derivatives thereof, such as for example the borohydride, cyanoborohydride, triacetoxyborohydride and trimethoxyborohydride salts: sodium, lithium, potassium, calcium and quaternary ammonium, such as tetramethylammonium, tetraethylammonium, tetra-n-butylammonium and benzyltriethylammonium, salts; catecholborane.

13. Method according to any one of Claims 1 to 6, in which the composition comprises an oxidizing agent,

14. Method according to any one of Claims 1 to 6, comprising a post-treatment with an oxidizing agent.

15. Method according to any one of Claims 1 to 6, comprising a step of conditioning post- treatment optionally combined with an oxidizing post- treatment.

16. Method according to either of Claims 13 and 14, in which the oxidizing agent is chosen from hydrogen peroxide, urea peroxide, alkali metal bromates, persalts such as perborates and persulfates, and enzymes.

17. Method according to any one of the preceding claims, in which the disulfide dye is present in a quantity of between 0.001 and 50% by weight, preferably between 0.005 and 20% by weight and more preferably still between 0.01 and 5% by weight relative to the total weight of the composition.

18. Method according to any one of the preceding claims, in which the composition contains at least one additional disulfide compound different from the compound of formula (I), (II), (III) or (IV).

19. Method according to the preceding claim, **characterized in that** the disulfide compound other than the compounds of formula (I), (II), (III) or (IV) is chosen from compounds comprising at least one fatty chain.

20. Method according to any one of the preceding claims, in which the composition comprises at least one oxidation base, one coupler and/or one direct dye other than a disulfide dye.

21. Dyeing composition comprising, in an appropriate cosmetic medium, a disulfide dye of formulae (I), (II), (III) or (IV) as defined above in Claims 1 to 6.

22. Composition according to Claim 21, comprising at least one oxidation base, One coupler and/or one direct dye other than a disulfide dye.

23. Composition according to other one of Claims 21 and 22, comprising at least one oxidizing agent.

24. Composition according to any one of Claims 21 to 23, comprising an organic solvent and/or a thickening agent.

25. Multicompartment device in which a first compartment contains a dyeing composition comprising a disulfide dye as defined in Claims 1 to 6, and a second compartment contains a reducing agent capable of reducing a disulphide bond.

26. Device according to Claim 25, comprising a third compartment which contains an oxidizing agent.

27. Use of the disulfide dyes as defined in Claims 1 to 6 for dyeing human keratin fibres, in particular hair.

28. Use according to Claim 27, for improving the fastness of the coloration.

29. Cationic disulfide dye comprising at least one quaternary ammonium radical of the following formulae (I), (II), (III) or (IV):
A-(X)ₚ-Cₛₐₜ-S-S-Cₛₐₜ-(X)ₚ-A (I) 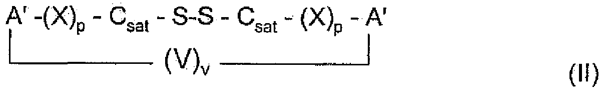 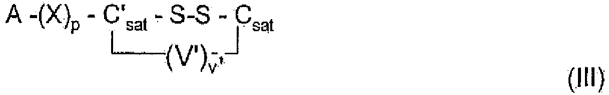
A-(X)ₚ-Cₛₐₜ-S-S-Cₛₐₜ-(X)ₚ-D (IV)
their salts, isomers and solvates such as hydrates, in which formulae:
• A and A', which are identical or different, represent a radical containing at least one chromophore; derived from cyanine-type dyes, said chromophores A and/or A' contain a cationic radical borne by or included in at least one of the chromophores; said cationic radical is a quaternary ammonium;
• V and V', which are identical or different, represent a bridging group;
• v and v', which are identical or different, represent 0 or 1;
• X represents the following sequence:
-(T)ₜ-(Y)_{y}-(Z)_{z}-
the said sequence being linked in formulae (I), (II), (III) or (IV) as follows:
-Cₛₐₜ(or C'ₛₐₜ)-(T)ₜ-(Y)_{y}-(Z)_{z}-(A or A'); in which **T** represents one or more radicals or combinations thereof, chosen from -SO₂-, -O-, -S-, -N(R)-, -N+(R)(R)-CO-, R representing a hydrogen atom, a C₁-C₄ alkyl radical or a C₁-C₄ hydroxyalkyl radical;
the coefficient **t** is equal to 0 or 1;
**Y** represents:
- a radical chosen from - (CH₂)₂-SO₂-; -CH₂-CHR-CO-NR'- with R, R', which are identical or different, representing a hydrogen atom, a C₁-C₄ alkyl radical.
- a group of formula (a), (a') or (a"): 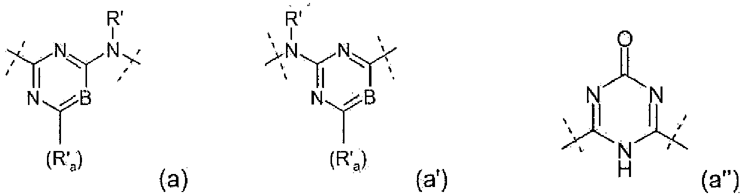 in which
• B represents -N-, -CRₐ, with Rₐ representing a hydrogen atom, a halogen atom chosen from chlorine or fluorine, a nitro group, a pyridinium group which is optionally substituted;
• R' has the same definition as above
• R'ₐ represents:
- a hydrogen atom
- a chlorine atom or a fluorine atom
- a pyridinium group which is optionally substituted with at least one group R_{c}, it being possible for R_{c} to be a C₁-C₄ alkyl group, a halogen atom, a carboxyl group -COOM (with M representing a hydrogen atom, an alkali metal, an ammonium group or an ammonium group substituted with one or more linear or branched, identical or different C₁-C₁₈ alkyl radicals, optionally bearing at least one hydroxyl); an ester group -COOR_{d} with R_{d} representing a C₁-C₄ alkyl radical; an amide group -CON(R_{d})₂ with R_{d}, which are identical or different, representing a hydrogen atom or a C₁-C₄ alkyl radical;
- a hydroxyl group
- an amino, alkylamino or dialkylamino group, the identical or different C₁-C₁₈ alkyl groups being linear or branched, optionally interrupted by a heteroatom chosen from N, 0, which are optionally substituted with one or more hydroxyl groups
- a group NHNHCOR where R represents a linear or branched C₁-C₁₀ alkyl group
- a group of the following formula (b): 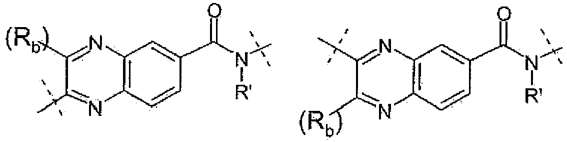 in which
• R' has the same definition as above
• R_{b} represents
- a chlorine atom
- an amino, alkylamino or dialkylamino group, the identical or different C₁-C₁₈ alkyl groups being linear or branched, optionally interrupted by a heteroatom chosen from N, 0, S, which are optionally substituted with one or more hydroxyls
- a saturated or unsaturated nitrogen-containing heterocycle which may be substituted
• an arylamino group in which the aryl radical is preferably C₆;
• the coefficient p is equal to 1;
• **y** is equal to 0 or 1;
• **Z** represents:
- - (CH₂)ₘ- with m integer between 1 and 8
- -(CH₂CH₂O)_{q}- or - (OCH₂CH₂)_{q}- in which q is an integer between 1 and 15
- an aryl, alkylaryl or arylalkyl radical whose alkyl radical is C₁-C₄ and the aryl radical is preferably C₆, being optionally substituted with at least one group SO₃M with M representing a hydrogen atom, an alkali metal or an ammonium ammonium group substituted with one or more identical or different, linear or branched C₁-C₁₈ alkyl radicals optionally bearing at least one hydroxyl
• **z** is equal to 0 or 1.
• Cₛₐₜ, C'ₛₐₜ, which are identical or different, represent an optionally substituted, optionally cyclic, linear or branched C₁-C₁₈ alkylene chain;
• D corresponds to a radical chosen from hydroxyl, hydroxyalkyl, alkoxy, carboxyl, carboxylate, amino, alkylamino and dialkylamino radicals it being understood that the compound of formula (I) cannot represent the following compounds:
